# EUROPEAN PATENT APPLICATION

(11) **EP 3 150 718 A1**
(43) Date of publication of application: **05.04.2017**
(21) Application number: 15800090.1
(22) Date of filing: 28.05.2015
(51) Int. Cl.: C12Q 1/68, G01N 33/574, G01N 33/53

(54) **METHOD FOR SENSITIVE DETECTION OF TARGET DNA USING TARGET-SPECIFIC NUCLEASE**

(30) Priority: 28.05.2014 KR 20140064526
(71) Applicant: Toolgen Incorporated, Seoul 08501 (KR); Institute for Basic Science, Daejeon 34047 (KR)
(72) Inventor: KIM, Jin Soo, Seoul 151-015 (KR); KIM, So Jung, Incheon 404-827 (KR); LEE, Seung Hwan, Seoul 132-788 (KR); KIM, Seok Joong, Seoul 137-911 (KR)
(74) Representative: Andrews, Robert
(86) International application number: PCT/KR2015/005383
(87) International publication number: WO 2015/183025

(57) **Abstract**

The present invention relates to a method for analyzing a genotype using a target-specific nuclease and, specifically, to a method for diagnosing cancer or analyzing a genotype by removing wild type DNA or particular genotype DNA using a target-specific nuclease or a variant thereof to amplify or concentrate only a small amount of DNA which has a difference in variation, such as a mutation, or genotype, and to a method for separating target DNA sesusing a target-specific nuclease or a variant thereof. Such methods are novel paradigm methods contrary to existing simple target-specific nucleases for post-PCR recognition of normal genotype and carcinogenic genotype, and can be favorably used in the early diagnosis of cancer or analysis of similar genotypes.

<110>    TOOLGEN INCORPORATED

<120>    A sensitive method for detecting target DNA using site-specific
         nuclease

<130>    OPA15082-PCT

<150>    KR 10-2014-0064526
<151>    2014-05-28

<160>    64

<170>    KopatentIn 2.0

<210>    1
<211>    23
<212>    DNA
<213>    Artificial Sequence

<220>
<223>    VEGFA_Off3


<400>    1
ggggagggga agtttgctcc tgg                                              23


<210>    2
<211>    23
<212>    DNA
<213>    Artificial Sequence

<220>
<223>    VEGFA_Off5


<400>    2
cgggggaggg agtttgctcc tgg                                              23


<210>    3
<211>    23
<212>    DNA
<213>    Artificial Sequence

<220>
<223>    VEGFA_Off27


<400>    3
ggaggagggg agtctgctcc agg                                              23


<210>    4
<211>    23
<212>    DNA
<213>    Artificial Sequence

<220>
<223>    VEGFA_Off16


<400>    4

ggtgggggtg ggtttgctcc tgg                                                23

<210>    5
<211>    23
<212>    DNA
<213>    Artificial Sequence

<220>
<223>    VEGFA_Off15

<400>    5
gagtgggtgg agtttgctac agg                                                23

<210>    6
<211>    23
<212>    DNA
<213>    Artificial Sequence

<220>
<223>    VEGFA_Off72

<400>    6
aagtaaggga agtttgctcc tgg                                                23

<210>    7
<211>    23
<212>    DNA
<213>    Artificial Sequence

<220>
<223>    VEGFA_Off3

<400>    7
ggggagggga agtttgctcc tgg                                                23

<210>    8
<211>    23
<212>    DNA
<213>    Artificial Sequence

<220>
<223>    VEGFA_Off5

<400>    8
ggaggagggg agtctgctcc agg                                                23

<210>    9
<211>    23
<212>    DNA
<213>    Artificial Sequence

<220>
<223>    VEGFA_Off27

<400>    9
ggtgggggtg ggtttgctcc tgg                                              23


<210>    10
<211>    23
<212>    DNA
<213>    Artificial Sequence

<220>
<223>    VEGFA_Off16


<400>    10
gagtgggtgg agtttgctac agg                                              23


<210>    11
<211>    23
<212>    DNA
<213>    Artificial Sequence

<220>
<223>    VEGFA_Off15


<400>    11
aggtggtggg agcttgttcc tgg                                              23


<210>    12
<211>    23
<212>    DNA
<213>    Artificial Sequence

<220>
<223>    VEGFA_Off56


<400>    12
gggcaagggg aggttgctcc tgg                                              23


<210>    13
<211>    23
<212>    DNA
<213>    Artificial Sequence

<220>
<223>    VEGFA_Off72


<400>    13
aagtaaggga agtttgctcc tgg                                              23


<210>    14
<211>    23
<212>    DNA
<213>    Artificial Sequence

<220>

<223>    FANCF

<400>    14
ggaatccctt ctgcagcacc tgg                                              23

<210>    15
<211>    101
<212>    RNA
<213>    Artificial Sequence

<220>
<223>    VEGF-A_Off3

<400>    15
ggggagggga agtttgctcc guuuuagagc uagaaauagc aaguuaaaau aaggcuaguc      60

cguuaucaac uugaaaaagu ggcaccgagu cggugcuuuu u                          101

<210>    16
<211>    101
<212>    RNA
<213>    Artificial Sequence

<220>
<223>    VEGF-A_Off5

<400>    16
ggaggagggg agtctgctcc guuuuagagc uagaaauagc aaguuaaaau aaggcuaguc      60

cguuaucaac uugaaaaagu ggcaccgagu cggugcuuuu u                          101

<210>    17
<211>    101
<212>    RNA
<213>    Artificial Sequence

<220>
<223>    VEGF-A_Off27

<400>    17
ggtgggggtg ggtttgctcc guuuuagagc uagaaauagc aaguuaaaau aaggcuaguc      60

cguuaucaac uugaaaaagu ggcaccgagu cggugcuuuu u                          101

<210>    18
<211>    101
<212>    RNA
<213>    Artificial Sequence

<220>
<223>    VEGF-A_Off16

<400>    18
gagtgggtgg agtttgctac guuuuagagc uagaaauagc aaguuaaaau aaggcuaguc      60

cguuaucaac uugaaaaagu ggcaccgagu cggugcuuuu u 101

<210> 19
<211> 102
<212> RNA
<213> Artificial Sequence

<220>
<223> VEGF-A_Off15

<400> 19
gaggtggtgg gagcttgttc cguuuuagag cuagaaauag caaguuaaaa uaaggcuagu 60

ccguuaucaa cuugaaaaag uggcaccgag ucggugcuuu uu 102

<210> 20
<211> 101
<212> RNA
<213> Artificial Sequence

<220>
<223> VEGF-A_Off56

<400> 20
gggcaagggg aggttgctcc guuuuagagc uagaaauagc aaguuaaaau aaggcuaguc 60

cguuaucaac uugaaaaagu ggcaccgagu cggugcuuuu u 101

<210> 21
<211> 102
<212> RNA
<213> Artificial Sequence

<220>
<223> VEGF-A_Off72

<400> 21
gaagtaaggg aagtttgctc cguuuuagag cuagaaauag caaguuaaaa uaaggcuagu 60

ccguuaucaa cuugaaaaag uggcaccgag ucggugcuuu uu 102

<210> 22
<211> 101
<212> RNA
<213> Artificial Sequence

<220>
<223> FANCF

<400> 22
ggaatccctt ctgcagcacc guuuuagagc uagaaauagc aaguuaaaau aaggcuaguc 60

cguuaucaac uugaaaaagu ggcaccgagu cggugcuuuu u 101

<210> 23
<211> 23
<212> DNA
<213> Artificial Sequence

<220>
<223> KRAS wild-type


<400> 23
aaacttgtgg tagttggagc tgg 23


<210> 24
<211> 103
<212> RNA
<213> Artificial Sequence

<220>
<223> KRAS wild-type


<400> 24
ggaaacttgt ggtagttgga gcguuuuaga gcuagaaaua gcaaguuaaa auaaggcuag 60

uccguuauca acuugaaaaa guggcaccga gucggugcuu uuu 103


<210> 25
<211> 23
<212> DNA
<213> Artificial Sequence

<220>
<223> KRAS wild-type


<400> 25
aaacttgtgg tagttggagc tgg 23


<210> 26
<211> 23
<212> DNA
<213> Artificial Sequence

<220>
<223> KRAS c.35G>A


<400> 26
aaacttgtgg tagttggagc tga 23


<210> 27
<211> 23
<212> DNA
<213> Artificial Sequence

<220>
<223> KRAS c.35G>T

<400>    27
aaacttgtgg tagttggagc tgt                                              23


<210>    28
<211>    23
<212>    DNA
<213>    Artificial Sequence

<220>
<223>    KRAS c.34G>T


<400>    28
aaacttgtgg tagttggagc ttg                                              23


<210>    29
<211>    23
<212>    DNA
<213>    Artificial Sequence

<220>
<223>    KRAS c.35G>C


<400>    29
aaacttgtgg tagttggagc tgc                                              23


<210>    30
<211>    23
<212>    DNA
<213>    Artificial Sequence

<220>
<223>    KRAS c.34G>C


<400>    30
aaacttgtgg tagttggagc tcg                                              23


<210>    31
<211>    20
<212>    DNA
<213>    Artificial Sequence

<220>
<223>    4134bp_sg#1


<400>    31
gagaaccaga ccacccagaa                                                  20


<210>    32
<211>    20
<212>    DNA
<213>    Artificial Sequence

<220>

<223>    4134bp_sg#2

<400>    32
ggcagccccg ccatcaagaa                                                    20

<210>    33
<211>    20
<212>    DNA
<213>    Artificial Sequence

<220>
<223>    2570bp_sg#1

<400>    33
gtaagatgct tttctgtgac                                                    20

<210>    34
<211>    20
<212>    DNA
<213>    Artificial Sequence

<220>
<223>    2570bp_sg#2

<400>    34
gatcctttga tcttttctac                                                    20

<210>    35
<211>    20
<212>    DNA
<213>    Artificial Sequence

<220>
<223>    1270bp_sg#1

<400>    35
gcctccaaaa aagaagagaa                                                    20

<210>    36
<211>    20
<212>    DNA
<213>    Artificial Sequence

<220>
<223>    1270bp_sg#2

<400>    36
tgacatcaat tattatacat                                                    20

<210>    37
<211>    20
<212>    DNA
<213>    Artificial Sequence

<220>
<223>    Exon1_sg#1


<400>    37
gggacacttt gcgttcgggc                                                    20


<210>    38
<211>    20
<212>    DNA
<213>    Artificial Sequence

<220>
<223>    Exon1_sg#2


<400>    38
aactctagag ccaccgtcca                                                    20


<210>    39
<211>    20
<212>    DNA
<213>    Artificial Sequence

<220>
<223>    Exon1_sg#3


<400>    39
agcgccagtc ttgagcacat                                                    20


<210>    40
<211>    20
<212>    DNA
<213>    Artificial Sequence

<220>
<223>    Exon2&3_sg#1


<400>    40
gatccactca cagtttccat                                                    20


<210>    41
<211>    20
<212>    DNA
<213>    Artificial Sequence

<220>
<223>    Exon2&3_sg#2


<400>    41
gtgggaagcg aaaattccat                                                    20


<210>    42
<211>    20

<212>    DNA
<213>    Artificial Sequence

<220>
<223>    Exon2&3_sg#3


<400>    42
ctcagagggg gctcgacgct                                                    20


<210>    43
<211>    20
<212>    DNA
<213>    Artificial Sequence

<220>
<223>    Exon4_sg#1


<400>    43
cttcccacag gtctctgcta                                                    20


<210>    44
<211>    20
<212>    DNA
<213>    Artificial Sequence

<220>
<223>    Exon4_sg#2


<400>    44
tggtgggcct gcccttccaa                                                    20


<210>    45
<211>    20
<212>    DNA
<213>    Artificial Sequence

<220>
<223>    Exon4_sg#3


<400>    45
cttccgggtc actgccatgg                                                    20


<210>    46
<211>    20
<212>    DNA
<213>    Artificial Sequence

<220>
<223>    Exon5_sg#1


<400>    46
agagttggcg tctacacctc                                                    20

<210>    47
<211>    20
<212>    DNA
<213>    Artificial Sequence

<220>
<223>    Exon5_sg#2


<400>    47
gaatcaaccc acagctgcac                                                    20


<210>    48
<211>    20
<212>    DNA
<213>    Artificial Sequence

<220>
<223>    Exon5_sg#3


<400>    48
cggcacccgc gtccgcgcca                                                    20


<210>    49
<211>    20
<212>    DNA
<213>    Artificial Sequence

<220>
<223>    Exon6_sg#1


<400>    49
ctcggataag atgctgagga                                                    20


<210>    50
<211>    20
<212>    DNA
<213>    Artificial Sequence

<220>
<223>    Exon6_sg#2


<400>    50
cacttttcga catagtgtgg                                                    20


<210>    51
<211>    20
<212>    DNA
<213>    Artificial Sequence

<220>
<223>    Exon6_sg#3


<400>    51
aaatttgcgt gtggagtatt                                                    20

<210>    52
<211>    20
<212>    DNA
<213>    Artificial Sequence

<220>
<223>    Exon7_sg#1


<400>    52
ggagtcttcc agtgtgatga                                                    20


<210>    53
<211>    20
<212>    DNA
<213>    Artificial Sequence

<220>
<223>    Exon7_sg#2


<400>    53
catgtagttg tagtggatgg                                                    20


<210>    54
<211>    20
<212>    DNA
<213>    Artificial Sequence

<220>
<223>    Exon7_sg#3


<400>    54
gcatgggcgg catgaaccgg                                                    20


<210>    55
<211>    20
<212>    DNA
<213>    Artificial Sequence

<220>
<223>    Exon8_sg#1


<400>    55
actgggacgg aacagctttg                                                    20


<210>    56
<211>    20
<212>    DNA
<213>    Artificial Sequence

<220>
<223>    Exon8_sg#2

<400>    56
gattctcttc ctctgtgcgc                                                    20


<210>    57
<211>    20
<212>    DNA
<213>    Artificial Sequence

<220>
<223>    Exon8_sg#3


<400>    57
ggtgaggctc ccctttcttg                                                    20


<210>    58
<211>    20
<212>    DNA
<213>    Artificial Sequence

<220>
<223>    Exon9_sg#1


<400>    58
gtgaaatatt ctccatccag                                                    20


<210>    59
<211>    20
<212>    DNA
<213>    Artificial Sequence

<220>
<223>    Exon9_sg#2


<400>    59
gggagaggag ctggtgttgt                                                    20


<210>    60
<211>    20
<212>    DNA
<213>    Artificial Sequence

<220>
<223>    Exon10_sg#1


<400>    60
tctcgaagcg ctcacgccca                                                    20


<210>    61
<211>    20
<212>    DNA
<213>    Artificial Sequence

<220>
<223>    Exon10_sg#2

<400>    61
gaactcaagg atgcccaggc                                                    20


<210>    62
<211>    20
<212>    DNA
<213>    Artificial Sequence

<220>
<223>    Exon11_sg#1


<400>    62
caatcagcca cattctaggt                                                    20


<210>    63
<211>    20
<212>    DNA
<213>    Artificial Sequence

<220>
<223>    Exon11_sg#2


<400>    63
   ctagaacttg acccccttga                                                    20


<210>    64
<211>    20
<212>    DNA
<213>    Artificial Sequence

<220>
<223>    Exon11_sg#3


<400>    64
gatgaaatcc tccagggtgt                                                    20

## Description

### [Technical Field]

The present invention relates to a method for analyzing a genotype using a target-specific nuclease, and specifically, the present invention relates to a method for diagnosing cancer or a method for analyzing a genotype by removing wild type DNA or particular genotype DNA using a target-specific nuclease or a variant thereof to amplify or concentrate a small amount of DNA, which has a difference in variation such as a mutation, or in genotype, and to a method for separating desired DNA using a target-specific nuclease or a variant thereof.

### [Background Art]

Blood plasma DNA has DNA fragments that are derived from many cells in the body. Although all human cells have the same genetic information, the human cells may become heterogeneous when external cells are applied (cell therapy or organ transplantation, etc.) or when mutations occur in internal cells.

Examples of such situations include: 1) cancer (cancer is caused by various mutations), 2) pregnancy (fetal DNA is not the same as the mother's DNA), 3) cell and organ transplantation (donor DNA is not the same as recipient DNA), etc. In each case, fragments of slightly different sequences for the same gene may be present in blood plasma DNA, even at a very low ratio. Therefore, the technique to sensitively observe the difference through a molecular diagnostic method is important.

In particular, since an excess amount of normal DNA and a small amount of cancer-derived DNA, in which mutations are induced, are mixed in a sample such as in the blood of a cancer patient, a method for analyzing the DNA is needed. However, existing diagnostic methods such as PCR-RFLP, etc. have been mainly used to amplify sample DNA through PCR and then detect the cleavage by cleaving the DNA with a restriction enzyme specific to a mutation (Hum. Mut. 2002 May; 21 (5): 535-41).Such methods have an advantage of the possibility of easily making a diagnosis in various cases, but there is still a disadvantage in that an early diagnosis of cancer results in false positives/negatives, etc. that are searched, and thus there is a limit to the accuracy of the information provided.

On the other hand, existing molecular diagnostic methods such as PCR or isothermal chain amplification (ICA), etc. have difficulties in analyzing genotypes of a similar species having similar sequences. For example, when a pathogenic bacterium/virus has a very similar sequence to a non-pathogenic bacterium/virus, a method for specifically detecting the genome of a different subject in the same species or in a similar species that has different sequences at specific sites, similar to native Korean cattle (Hanwoo) and imported cattle, is required. If existing molecular diagnostic methods do not completely distinguish similar sequences, false positives/negatives are likely to occur.

### [Disclosure]

### [Technical Problem]

As a result of intensive efforts to find a more accurate method for diagnosis and analyzing a genotype, the present inventors have surprisingly confirmed that, contrary to the paradigm of existing diagnostic or genotyping methods, a small amount of desired DNA having a difference in variation such as a mutation, or in genotype can be concentrated by removing undesired DNA by cleaving the undesired DNA while masking the desired DNA from cleavage using a guide RNA specific to the desired DNA and an inactivated Cas9 protein complex, or by primarily removing the undesired DNA by cleaving the undesired DNA using a target-specific nuclease. Further, it was confirmed that a small amount of desired DNA can be concentrated by isolating and purifying the desired DNA using a guide RNA specific to the desired DNA and an inactivated Cas9 protein complex. In particular, it was confirmed that the desired DNA can be isolated in a non-covalent bonding, using the inactivated Cas protein and the guide RNA targeting cell-derived DNA. The present invention was completed by confirming that the concentrated desired DNA can be used in various fields such as diagnosis of cancer, prediction of the prognosis for cancer, diagnosis of pregnancy, and cell/organ transplantation, by molecular detection or separation and purification.

### [Technical Solution]

An object of the present invention is to provide a method for analyzing a genotype comprising: (i) removing particular genotype DNA in an isolated sample, in which two or more genotypes of DNA are mixed, by cleaving the particular genotype DNA using a nuclease specific to the particular genotype DNA; and (ii) analyzing other DNA present in the sample in which the particular genotype DNA has been removed.

Another object of the present invention is to provide a method for analyzing a genotype comprising: (i) removing other genotype DNA by cleaving the other genotype DNA while masking particular genotype DNA in an isolated sample, in which two or more genotypes of DNA are mixed, for protection from cleavage by RGEN capable of recognizing the other genotype DNA, using a guide RNA specific to the particular genotype DNA and inactivated Cas nuclease protein; and (ii) analyzing the particular genotype DNA in the sample in which the other genotype DNA has been removed.

Another object of the present invention is to provide a method for analyzing a genotype of DNA in an isolated sample comprising: (i) removing DNA of a non-pathogenic bacterium or virus from the sample by cleaving the DNA of the non-pathogenic bacterium or virus by treating a nuclease specific to the DNA of the non-pathogenic bacterium or virus in the sample having DNA of bacteria or viruses.; and (ii) analyzing DNA of a pathogenic bacterium or virus in the sample in which the DNA of the non-pathogenic bacterium or virus has been removed.

Another object of the present invention is to provide a method for analyzing a genotype of DNA in a isolated sample comprising: (i) removing DNA of a non-pathogenic bacterium or virus in the sample by cleaving the DNA of the non-pathogenic bacterium or virus while masking DNA of a pathogenic bacterium or virus for protection from cleavage by RGEN specific to the non-pathogenic bacterium or virus, using a guide RNA specific to the DNA of the pathogenic bacterium or virus and an inactivated Cas nuclease protein (dCas); and (ii) analyzing the DNA of the pathogenic bacterium or virus in the sample in which the DNA of the non-pathogenic bacterium or virus has been removed.

Another object of the present invention is to provide a method for separating desired DNA from an isolated sample containing two or more genotypes of DNA using an inactivated nuclease specific to the desired DNA.

Another object of the present invention is to provide a method for separating desired DNA from an isolated sample containing genomic DNA using an inactivated nuclease specific to the desired DNA.

Another object of the present invention is to provide a method for analyzing a genotype comprising: (i) separating particular genotype DNA in an isolated sample, in which two or more genotypes of DNA are mixed using an inactivated nuclease specific to the particular genotype DNA; and (ii) analyzing the separated particular genotype DNA.

### [Advantageous Effects]

The present invention, which is based on a concept contrary to the existing methods for diagnosis or analysis of a genotype, provides a method for diagnosing cancer or analyzing a genotype by concentrating desired DNA, by (i) cleaving undesired DNA, for example, normal DNA, using a target-specific nuclease such as ZFN, TALEN, and RGEN before amplifying the desired DNA in a sample, or (ii) separating desired DNA, using an inactivated Cas9:gRNA complex. In the methods of the present invention, by going through a process of concentrating the desired DNA before the detection thereof, false positive/negative signals, etc. are removed, which makes an early diagnosis of cancer possible, and a method of a new paradigm for accurate diagnosis for the prognosis of a cancer patient is provided. Further, through this process, an early diagnosis of cancer cells, prediction for the prognosis of cancer, and monitoring for the occurrence of metastatic cancer after surgery and chemotherapy are possible.

### [Brief Description of the Drawings]

FIG. 1 shows a schematic diagram of two methods according to the present invention for concentrating target DNA using RGEN.
FIG. 2 shows a schematic diagram of a method for increasing the mutation rates induced by gene scissors.
FIG. 3 confirms changes in the mutation rates in a genomic DNA mixture having a mutation rate of 0.0054% to 54%, before and after cleavage.
FIG. 4 shows a schematic diagram illustrating a method for confirming whether or not it is possible to perform a diagnosis for mutations in an oncogene by applying the new paradigm of the present invention.
FIG. 5 shows the development of RGEN for RFLP for detecting oncogene mutations and the results.
FIG. 6 shows a graph illustrating the results confirmed by using RGEN for RFLP for detecting oncogene mutations.
FIG. 7 shows a schematic diagram illustrating the mutant gene amplification and the detection process using CUT-PCR.
FIG. 8 shows a graph illustrating the results of experiments for detecting a KRAS mutant gene using plasmids. The right-hand diagram shows plasmids containing wild type and mutant KRAS. WT: wild type normal gene;MT: mutant gene.
FIG. 9 shows a graph illustrating the ratio of normal and mutant KRAS genes (left) and the rate of increase of mutant KRAS gene (right).

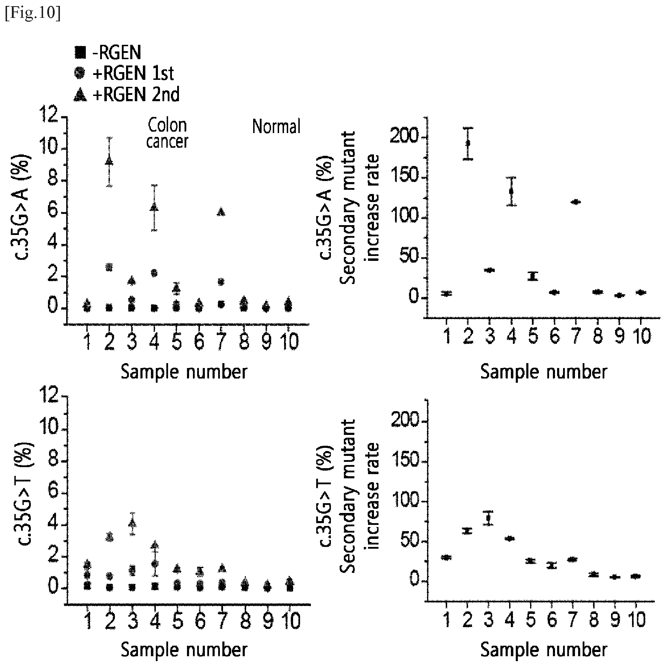

FIG. 10 shows a graph illustrating the results of two rounds of performing CUT-PCR using cell-free DNA obtained from blood plasma of colon cancer patients and normal individuals at various stages of progression. In each of cell-free DNAs obtained from the blood plasma, by targeting c.35G>A and c.35G>T mutant genes of KRAS having a high frequency of occurrence, the number of mutant genes (%) amplified in the first and second rounds was shown. RGEN 1^{st}: RGEN specific to normal gene was treated once, and PCR was performed; RGEN 2^{nd}: RGEN specific to normal gene was treated twice, and PCR was performed.
FIG. 11 shows a schematic diagram illustrating a procedure of a target DNA fragment purification experiment using dCas9.

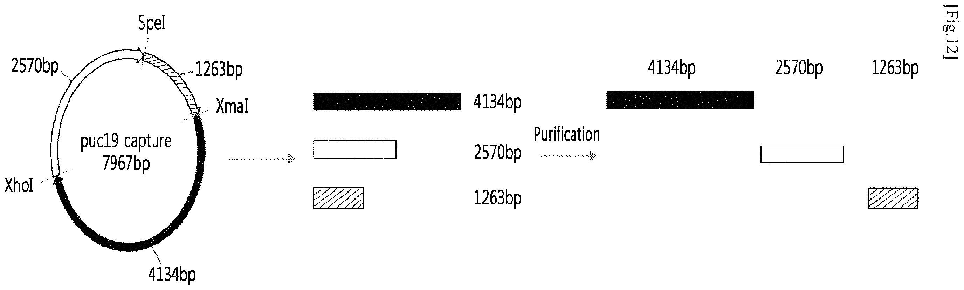

FIG. 12 shows a schematic diagram illustrating an experimental procedure for purifying a plasmid fragment using dCas9.
FIG. 13 shows the results of purifying each target DNA with one sgRNA or two sgRNAs. Input is a sample of pre-purified plasmid DNA cleaved with a restriction enzyme.
FIG. 14 shows a diagram illustrating the results of purifying two target DNAs out of three target DNAs. Input is a sample of pre-purified plasmid DNA cleaved with a restriction enzyme.
FIG. 15 shows a graph illustrating the intensity (%) of an agarose gel band of each target DNA observed after purification of the target DNA.
FIG. 16 shows a graph illustrating the results of performing purification of the TP53 gene exons of HeLa cells and SW 480 cells and performing Real-Time qPCR.
FIG. 17 shows a schematic diagram illustrating a method for diagnosis and identifying the DNA of a pathogenic bacterium having a similar sequence.

### [Best Mode]

As one aspect to achieve the above objects, the present invention provides a method for analyzing other genotype DNA by removing particular genotype DNA in an isolated sample in which two or more genotypes of DNA are mixed.

Specifically, the method for analyzing a genotype may be performed comprising: (i) removing particular genotype DNA in an isolated sample, in which two or more genotypes of DNA are mixed, by cleaving the particular genotype DNA using a nuclease specific to the particular genotype DNA; and (ii) analyzing desired other genotype DNA.

The other genotype DNA can be detected by amplification by PCR or other methods known in the art.

Specifically, other genotype DNA can be analyzed using PCR, sequencing (e.g., deep sequencing, Sanger sequencing, and NGS), and RFLP using a target-specific nuclease (e.g., RGEN RFLP).

The above-described "remove" includes the concept that particular genotype DNA cannot be amplified by PCR, etc. because the particular genotype DNA has been cleaved, and includes the concept of complete or partial removal in a sample.

More specifically, the method may provide a method for analyzing a genotype of DNA that may be performed comprising (a) removing particular genotype DNA in an isolated sample, in which two or more genotypes are mixed, by cleaving the particular genotype DNA using a nuclease specific to the particular genotype DNA; (b) amplifying other genotype DNA present in a sample in which the particular genotype DNA has been removed; and (c) analyzing the amplified other genotype DNA.

Further, when the other genotype DNA is DNA derived from cancer, the method for analyzing a genotype may be a method for analyzing a genotype for providing information for diagnosing cancer.

Specifically, the method for diagnosing cancer may be a method for providing information for diagnosing cancer, comprising: step (a), in which particular genotype DNA is wild type DNA, of removing the wild type DNA in an isolated sample by cleaving the wild type DNA using a nuclease specific to the wild type DNA; step (b), in which other genotype DNA is DNA having a cancer-specific mutation, of amplifying the DNA having the cancer-specific mutation in the sample in which the wild type DNA has been removed; and step (c) of analyzing the sequence.

The present inventors completed the present invention by confirming that when the new method is used, which is contrary to the paradigm of existing diagnostic methods or existing methods for analyzing a genotype, which comprises 1) removing normal DNA in a sample by cleaving the normal DNA with a nuclease specific to the normal DNA, and 2) amplifying only a small amount of cancer-derived DNA and performing RFLP or sequence analysis, it was possible to make a diagnosis without false positives/false negatives. The concept of cleaving and removing normal DNA rather than mutant DNA before amplification by PCR is a concept originally developed by the present inventors.

As used herein, the term "target-specific nuclease" is a nuclease capable of recognizing and cleaving a specific site of DNA in a target genome. The nuclease may comprise a nuclease in which a cleavage domain and a domain that recognizes a specific target sequence in the genome are fused, for example, a meganuclease; a fusion protein in which a cleavage domain and a transcription activator-like effector (TAL) domain, which is a transcription activator-like effector nuclease (TALEN) derived from a plant pathogenic gene, capable of recognizing a specific target sequence in the genome, are fused; a zinc finger nuclease; or an RNA-guided engineered nuclease (RGEN), but is not limited thereto. For the purposes of the present invention, the method using RGEN can achieve simple yet more favorable results.

As another embodiment of the present invention, the nuclease may be a zinc finger nuclease (ZFN). ZFN comprises a zinc finger protein engineered to bind to a selected gene and to a target site in a cleavage domain or a cleavage half-domain. The zinc finger binding domain can be engineered to bind to a selected sequence. For example, Beerli et al. (2002) Nature Biotechnol. 20: 135-141; Pabo et al. (2001) Ann. Rev. Biochem. 70: 313-340; Isalan et al., (2001) Nature Biotechnol. 19: 656-660; Segal et al. (2001) Curr. Opin. Biotechnol. 12: 632-637; and Choo et al. (2000) Curr. Opin. Struct. Biol. 10: 411-416. may be referenced. Compared to naturally occurring zinc finger proteins, the engineered zinc finger binding domain can have novel binding specificities. The method of operation includes rational design and selection of various types, but is not limited thereto. The rational design includes the use of databases containing, for example, triple (or quadruple) nucleotide sequences and individual zinc finger amino acid sequences, wherein each triple or quadruple nucleotide sequence is combined with one or more sequences of a zinc finger that binds to a particular triple or quadruple sequence.

Selection of target sequences and design and construction of the fusion protein (and a polynucleotide encoding the fusion protein) are well known to those skilled in the art and are described in detail in U.S. Patent Application Publication Nos. 2005/0064474 and 2006/0188987, the entire contents of which are incorporated herein by reference. Further, as disclosed in these and other references, zinc finger domains and/or multi-finger zinc finger proteins may be linked by a linker comprising any appropriate linker sequence, for example, a linker 5 or more amino acids in length. Examples of linker sequences 6or more amino acids in length are disclosed in U.S. Patent Nos. 6,479,626,6,903,185,and 7,153,949. The proteins described herein may comprise any combination of appropriate linkers between each zinc finger of the protein.

Further, a nuclease such as ZFN and/or a meganuclease comprises a cleavage domain or a cleavage half-domain. As noted above, the cleavage domain may be heterologous to the DNA binding domain, such as, for example, a cleavage domain from a nuclease which includes different type of zinc finger DNA binding domain, a cleavage domain from a nuclease which includes different type of meganuclease DNA binding domain. The heterologous cleavage domain may be obtained from any endonuclease or exonuclease. An exemplary endonuclease, from which the cleavage domain may be derived, may comprise a restriction endonucleaseor a meganuclease, but is not limited thereto.

Similarly, a cleavage half-domain may be derived from, as indicated above, any nuclease or a portion thereof that requires dimerization for cleavage activity. When a fusion protein comprises a cleavage half-domain, generally two fusion proteins are required for cleavage. Alternatively, a single protein comprising two cleavage half-domains may be used. The two cleavage half-domains may be derived from the same endonuclease (or functional fragments thereof), or each cleavage half-domain may be derived from a different endonuclease (or functional fragments thereof). Further, the target site of the two fusion proteins is preferred to be positioned such that the cleavage half domain can form functional cleavage domains by, for example, dimerization, with the cleavage half domains being positioned spatially orientated to each other by the binding of the two fusion proteins and their respective target sites. Thus, in one embodiment, the neighboring edges of the target site of 5 to 8 nucleotides or 15 to 18 nucleotides are separated. However, any integer number of nucleotides or nucleotide pairs may be interposed between the two target sites (*e.g.,* 2 to 50 nucleotide pairs or more). Generally, the cleavage site lies between the target sites.

Restriction endonucleases (restriction enzymes) are present in many species and can sequence-specifically bind to DNA (at a target site) to cleave DNA directly at or around the binding site. Some restriction enzymes (*e.g*., Type IIS) cleave DNA at sites distant from a recognition site, and have separable binding and cleavable domains. For example, Type II enzyme FokI catalyzes the double strand cleavage at 9 nucleotides from a recognition site on one strand and at 13 nucleotides from a recognition site on the other strand. Thus, in one embodiment, the fusion protein comprises a cleavage domain (or a cleavage half-domain) of at least 1 Type IIS restriction enzyme and one or more zinc finger binding domains (which may or may not be engineered).

As used herein, the term "TALEN" means a nuclease capable of recognizing and cleaving a target region of DNA. TALEN refers to a fusion protein comprising a TALE domain and a nucleotide cleavage domain. In the present invention, the terms "TAL effector nuclease" and "TALEN" are interchangeable. The TAL effector is a protein secreted by the Type III secretion system of *Xanthomonas* bacteria, when a variety of plant species are infected by the *Xanthomonas* bacteria. The protein may bind to a promoter sequence in a host plant to activate the expression of a plant gene that aids in bacterial infection. The protein recognizes plant DNA sequences through a central repeating domain comprising a variable number of amino acid repeats up to 34. Thus, TALE may be a new platform for tools in genome engineering. However, in order to prepare functional TALEN with genome-editing activity, a few key parameters that have not been known to date should be defined as follows:
(i) the minimum DNA-binding domain of TALE (ii) the length of the spacer between two half-spaces constituting one target region, and (iii) the linker connecting the FokI nuclease domain to dTALE or a fusion junction.

A TALE domain of the present invention refers to a protein that binds to a nucleotide in a sequence-specific manner via one or more TALE-repeat modules. The TALE domain comprises at least one TALE-repeat module, preferably 1 to 30 TALE-repeat modules, but is not limited thereto. In the present invention, the terms "TAL effector domain" and "TALE domain" are interchangeable. The TALE domain may comprise half of a TALE-repeat module.

As used herein, the term "RGEN" means a nuclease composed of a nuclease specific to target DNA and a Cas protein.

As used herein, the term "Cas protein" means a major protein component of the CRISPR/Cas system and forms a complex with a CRISPR RNA (crRNA) and a trans-activating crRNA (tracrRNA) to form an activated endonuclease or a nickase.

The Cas protein may be, but is not limited to, a Cas9 protein. Further, the Cas9 protein may be derived from *Streptococcus pyogenes,* but is not limited thereto.

A Cas protein or genetic information may be obtained from known databases such as GenBank of National Center for Biotechnology Information (NCBI), but is not limited thereto.

A Cas protein may be linked to a protein transduction domain. The protein transduction domain may be a poly-arginine or an HIV-derived TAT protein, but is not limited thereto.

A Cas protein may be linked to a tag which is advantageous for separation and/or purification depending on the object. Examples of the tag that may be linked comprise a His tag, a Flag tag, an S tag, a glutathione S-transferase (GST) tag, a maltose binding protein (MBP) tag, a chitin binding protein (CBP) tag, an Avi tag, a calmodulin tag, a polyglutamate tag, an E tag, an HA tag, an myc tag, an SBP tag, softag 1, softag 3, a strep tag, a TC tag, an Xpress tag, a biotin carboxyl carrier protein (BCCP) tag, or a green fluorescent protein (GFP) tag, etc. depending on the object, but are not limited thereto.

As used herein, the term "guide RNA" refers to RNA specific to target DNA, which may be combined with a Cas protein to lead the Cas protein to target DNA. The target DNA may be used interchangeably with desired DNA.

In the present invention, a guide RNA may be composed of two RNAs, *i.e.,* a CRISPR RNA (crRNA) and a trans-activating crRNA (tracrRNA). Or the guide RNA may be a single-chain RNA (sgRNA) prepared by fusion of major parts of a crRNA and a tracrRNA.

A guide RNA may be a dualRNA comprising a crRNA and a tracrRNA.

A crRNA may bind to target DNA.

RGEN may be composed of a Cas protein and a dualRNA, or may be composed of a Cas protein and an sgRNA. A guide RNA may comprise one or more additional nucleotides at the 5' end of a crRNA of a sgRNA or a dualRNA.

A guide RNA may be delivered into a cell in the form of either an RNA or a DNA encoding the RNA.

The method for diagnosing cancer may be an early diagnosis of cancer.

The method for diagnosing cancer may be a method for predicting the prognosis of cancer.

The DNA having a mutation may be derived from a cancer cell.

An isolated sample in which two or more genotypes of DNA are mixed may be a blood sample isolated from a subject suspected of having cancer.

The method for analyzing a genotype may be used for providing information for predicting the prognosis of a subject that has received cell or organ transplantation wherein: step (i), in which particular genotype DNA is the DNA of a subject that has received cell or organ transplantation, is to remove the DNA of the subject that has received cell or organ transplantation in a sample, in which two or more genotypes of DNA are mixed, isolated from the subject that has received cell or organ transplantation, by cleaving the DNA of the subject using a nuclease specific to the DNA of the subject; and step (ii), in which other genotype DNA is the DNA of a transplanted cell or organ, comprises analyzing the DNA of the transplanted cell or organ in the sample in which the DNA of the subject has been removed.

The method for analyzing a genotype may be used for collecting information regarding a crime scene wherein: step (i), in which particular genotype DNA is the DNA of a victim, is to remove the DNA of the victim in a DNA sample, in which two or more genotypes of DNA are mixed, derived from the crime scene by cleaving the DNA of the victim using a nuclease specific to the DNA of the victim; and step (ii), in which other genotype DNA is the DNA of an assailant, comprises analyzing the DNA of the assailant in the sample in which the DNA of the victim has been removed; or the method may comprise: step (i), in which particular genotype DNA is the DNA of an assailant, of removing the DNA of the assailant in a DNA sample, in which two or more genotypes of DNA are mixed, derived from the crime scene, by cleaving the DNA of the assailant using a nuclease specific to the DNA of the assailant; and step (ii), in which other genotype DNA is the DNA of the victim, which comprises analyzing the DNA of the victim in a sample in which the DNA of the assailant has been removed.

As another aspect, the present invention provides a method for analyzing a genotype using a guide RNA specific to particular genotype DNA and an inactivated nuclease protein (dCas).

Specifically, the method for analyzing a genotype may comprise: (i) removing other genotype DNA by cleaving the other genotype DNA while masking particular genotype DNA in an isolated sample, in which two or more genotypes of DNA are mixed, for protection from cleavage by RGEN capable of recognizing the other genotype DNA using a guide RNA (gRNA) specific to the particular genotype DNA and an inactivated Cas nuclease protein; and (ii) analyzing the particular genotype present in the sample in which the other genotype DNA has been removed.

More specifically, the method provides a method for analyzing a genotype that may be performed comprising: (a) removing other genotype DNA by cleaving the other genotype DNA while masking the particular genotype DNA in an isolated sample, in which two or more genotypes of DNA are mixed, for protection from cleavage by RGEN capable of recognizing the other genotype DNA using a guide RNA specific to the particular genotype DNA and an inactivated Cas nuclease protein; (b) amplifying the particular genotype DNA in the sample in which the other genotype DNA has been removed; and (c) analyzing the amplified particular genotype DNA.

Further, when the other genotype DNA is DNA derived from cancer, the method for analyzing a genotype may be a method for analyzing a genotype for providing information for diagnosing cancer.

Specifically, the method for diagnosing cancer may be a method for providing information for diagnosing cancer, comprising: step (a), in which other genotype DNA is wild type DNA, and particular DNA is DNA having a cancer-specific mutation, of removing the wild type DNA in an isolated sample by cleaving the wild type DNA while masking the particular genotype DNA for protection from cleavage by RGEN specific to the wild type DNA, using an inactivated RGEN (a dCas9:gRNA complex) composed of a guide RNA capable of specifically binding to the particular DNA having the mutation and an inactivated Cas nuclease protein (dCas); step (b) of amplifying the DNA having the cancer-specific mutation in the sample in which the wild type DNA has been removed; and step (c) of analyzing the amplified DNA having the mutation.

The method for diagnosing cancer may be an early diagnosis of cancer.

The method for diagnosing cancer may be a method for predicting the prognosis of cancer.

The DNA having a mutation may be derived from cancer.

The isolated sample in which two or more genotypes of DNA are mixed may be derived from a subject suspected of having cancer. Specifically, it may be an isolated blood sample containing a cfDNA or cfDNA sample.

As used herein, the term "inactivated RGEN" means RGEN comprising a Cas nuclease protein in which all or part of the function of the nuclease is inactivated. The inactivated Cas protein is also named as dCas. The Cas protein may be a Cas9 protein. The preparation of the inactivated Cas9 nuclease protein comprises, but is not limited to, any method by which the activity of the nuclease is inactivated, for example, by introducing D10A andH840A variations into the Cas9 nuclease protein.D10A Cas9 and H840A Cas9 nucleases proteins, that is, Cas9 nuclease proteins, which are each prepared by introducing a variation at only one of the active sites present in the Cas9 nuclease protein, can function as a nickase when binding to a guide RNA.

Such nickase is included in the category of RGEN because it may cause a double strand breakage (DBS) by cleaving both DNA strands on both sides when using two nickases.

In another example, by introducing all of the D10A and H840A mutations to a Cas9 nuclease protein, D10A/H840A Cas9 proteins, that is, dCas9 proteins, which are each prepared by introducing mutations at the two active sites of the Cas9 nuclease, can function as DNA binding complexes, which do not cleave DNA when binding with a guide DNA.

The method for analyzing a genotype can be used for providing information for predicting the prognosis of a subject that has received cell or organ transplantation wherein: step (i), in which particular genotype DNA is the DNA of a subject that has received cell or organ transplantation, is to remove the DNA of the subject in a sample, in which two or more genotypes of DNA are mixed, isolated from the subject that has received cell or organ transplantation, by cleaving the DNA of the subject using a nuclease specific to the DNA of the subject; and step (ii), in which other genotype DNA is the DNA of a transplanted cell or organ, comprises analyzing the DNA of the transplanted cell or organ in the sample in which the DNA of the subject has been removed.

The method for analyzing a genotype can be used for collecting information regarding a crime scene wherein: step (i), in which particular genotype DNA is the DNA of a victim, is to remove the DNA of the victim in a DNA sample, in which two or more genotypes of DNA are mixed, derived from the crime scene by cleaving the DNA of the victim using a nuclease specific to the DNA of the victim; and step (ii), in which other genotype DNA is the DNA of an assailant, comprises analyzing the DNA of the assailant in the sample in which the DNA of the victim has been removed; or the method may comprise: step (i), in which particular genotype DNA is the DNA of an assailant, of removing the DNA of the assailant in a DNA sample, in which two or more genotypes of DNA are mixed, derived from the crime scene, by cleaving the DNA of the assailant using a nuclease specific to the DNA of the assailant; and step (ii), in which other genotype DNA is the DNA of the victim, which comprises analyzing the DNA of the victim in the sample in which the DNA of the assailant has been removed.

As another aspect, the present invention provides a method for analyzing a genotype of DNA in an isolated sample comprising: (i) removing the DNA of a non-pathogenic bacterium or virus in the sample by cleaving the DNA of the non-pathogenic bacterium or virus by treating the DNA of the non-pathogenic bacterium or virus with a nuclease specific to the DNA of the non-pathogenic bacterium or virus, in the sample having DNA of bacteria or viruses; and (ii) analyzing the DNA of a pathogenic bacterium or virus in the sample in which the DNA of the non-pathogenic bacterium or virus has been removed.

Specifically, the present invention provides a method for analyzing a genotype of DNA in an isolated sample that may be performed comprising: (a) removing the DNA of a non-pathogenic bacterium or virus in the sample by cleaving the DNA of the non-pathogenic bacterium or virus by treating the DNA of the non-pathogenic bacterium or virus with a nuclease specific to the DNA of the non-pathogenic bacterium or virus, in the sample having DNA of bacteria or viruses; (b) amplifying the DNA of a pathogenic bacterium or virus in the sample in which the DNA of the non-pathogenic bacterium or virus has been removed; and (c) analyzing the amplified DNA of the pathogenic bacterium or virus.

The nuclease may be selected from the group consisting of a zinc finger nuclease (ZFN), a transcription activator-like effector nuclease (TALEN), and an RNA-guided engineered nuclease (RGEN), but is not limited thereto.

As another aspect, the present invention provides a method for analyzing a genotype of DNA in an isolated sample comprising: (i) removing the DNA of a non-pathogenic bacterium or virus in the sample by cleaving the DNA of the non-pathogenic bacterium or virus while masking the DNA of a pathogenic bacterium or virus for protection from cleavage by RGEN specific to the DNA of the non-pathogenic bacterium or virus using a guide RNA specific to the pathogenic bacterium or virus and inactivated RGEN (a dCas9:gRNA complex) composed of an inactivated Cas9 nuclease protein; and (ii) analyzing the DNA of the pathogenic bacterium or virus in the sample in which the DNA of the non-pathogenic bacterium or virus has been removed.

Specifically, the method provides a method for analyzing a genotype of DNA in an isolated sample that may be performed comprising: (a) removing the DNA of a non-pathogenic bacterium or virus in the sample by cleaving the DNA of the non-pathogenic bacterium or virus while masking the DNA of a pathogenic bacterium or virus for protection from cleavage by RGEN specific to the DNA of the non-pathogenic bacterium or virus using a guide RNA specific to the DNA of the pathogenic bacterium or virus and inactivated RGEN (a dCas9:gRNA complex) composed of an inactivated Cas9 nuclease protein; (b) amplifying the DNA of the pathogenic bacterium or virus in the sample in which the DNA of the non-pathogenic bacterium or virus has been removed; and (c) analyzing the amplified DNA of the pathogenic bacterium or virus.

As another aspect, the present invention provides a method for separating desired DNA, comprising separating the desired DNA in an isolated sample containing two or more types of DNA, using an inactivated nuclease specific to the desired DNA.

Specifically, the method for separating the desired DNA may be performed comprising: forming a dCas-gRNA-desired DNA complex from a guide RNA (gRNA) capable of specifically binding to the desired DNA, an inactivated Cas protein (dCas), and the desired DNA; and separating the complex from the sample.

The desired DNA can be detected by amplification by PCR or by known methods.

The method for separating may be applied to cell-free DNA *in vitro* and may be performed without forming a cross-link covalent bond between the DNA, the gRNA, and the dCas protein.

The method for separating may further comprise separating the desired DNA from the complex.

In order to separate the desired DNA, the inactivated Casprotein may comprise an affinity tag for separation, for example, the affinity tag may be a His tag, a Flag tag, an S tag, a glutathione S-transferase (GST) tag, a maltose binding protein (MBP) tag, a chitin binding protein (CBP) tag, an Avi tag, a calmodulin tag, a polyglutamate tag, an E tag, an HA tag, an myc tag, an SBP tag, softag 1, softag 3, a strep tag, a TC tag, an Xpress tag, a biotin carboxyl carrier protein (BCCP) tag, or a green fluorescent protein (GFP) tag, but is not limited thereto.

The inactivated Cas protein may lack the DNA cleavage activity of Cas proteins, and specifically, the inactivated Cas protein may be a variant of a Cas9 protein having a D10A, H840A, or a D10A/H840A mutation, but is not limited thereto.

The Cas9 protein may be derived from *Streptococcus pyogenes.*

The method may be for separating desired DNA using an affinity column or a magnetic bead binding to the tag. For example, an affinity tag for the separation may be a His tag, and the desired DNA may be separated using a metal affinity column or a magnetic bead that binds to the His tag, and the magnetic bead may be, for example, a Ni-NTA magnetic bead, but is not limited thereto.

The separation of desired DNA from the complex may be performed using an RNase and a protease.

Using the method of separating desired DNA, particular genotype DNA can be separated in an isolated sample in which two or more genotypes of DNA are mixed, and two or more types of desired DNA can be separated. When two or more types of desired DNA are separated, the desired DNA can be separated using a guide RNA specific to each type of the desired DNA.

The guide RNA may be a single-chain guide RNA (sgRNA), or may be a dualRNA comprising a crRNA and a tracrRNA. Further, the guide RNA may be in a form of separated RNA, or may be in an encoded form in a plasmid.

As another aspect, the present invention provides a method for separating desired DNA comprising: separating the desired DNA in an isolated sample containing genomic DNA using an inactivated nuclease specific to the desired DNA.

Specifically, the method may be performed comprising: forming a dCas-gRNA-desired DNA complex from a guide RNA (gRNA) capable of specifically binding to the desired DNA, an inactivated Cas protein (dCas), and the desired DNA; and separating the complex from the sample.

The constitution of each step of the method for separating the desired DNA is as described above.

As another aspect, the present invention provides a method for analyzing a genotype comprising: (i) removing particular genotype DNA in an isolated sample, in which two or more genotypes of DNA are mixed, by cleaving the particular genotype DNA using a nuclease specific to the particular genotype DNA; and (ii) analyzing the separated particular genotype DNA.

Specifically, the method may be performed comprising: (a) separating particular genotype DNA in an isolated sample, in which two or more genotypes of DNA are mixed, by cleaving the particular genotype DNA using a nuclease specific to the particular genotype DNA; (b) amplifying the separated particular genotype DNA; and (c) analyzing the amplified particular genotype DNA.

Further, when particular genotype DNA is DNA derived from cancer, the method for analyzing a genotype may be a method for analyzing a genotype for providing information for diagnosing cancer.

Specifically, the method for diagnosing cancer may be a method for providing information for diagnosing cancer by analyzing the particular genotype DNA, in which the particular genotype DNA is DNA having a cancer-specific mutation.

The present inventors completed the present invention by confirming that after purifying particular genotype DNA using an inactivated nuclease specific to the particular genotype, it was possible to make a diagnosis without false positives/false negatives when only a small amount of cancer-derived DNA was purified, and RFLP or sequence analysis were performed.

The nuclease may be, for example, a zinc finger nuclease (ZFN), a transcription activator-like effector nuclease (TALEN), or an RNA-guided engineered nuclease (RGEN), but is not limited thereto.

The method for diagnosing cancer may be an early diagnosis of cancer, or may be a method for predicting the prognosis of cancer.

The method may comprise, specifically: (i) forming a dCas-gRNA-particular genotype DNA complex by treating the sample with a guide RNA (gRNA) capable of specifically binding to the particular genotype DNA and an inactivated Cas protein (dCas); and (ii) analyzing the particular genotype DNA separated from the complex.

The inactivated Cas protein may comprise an affinity tag for separation, examples of which are described above.

The inactivated Cas protein may lack the cleavage activity of Cas proteins, as described above.

The method may be for separating particular genotype DNA using an affinity column or a magnetic bead binding to the tag.

The separation of particular genotype DNA from the complex may be performed using an RNase and a protease.

### [Mode of Invention]

Hereinafter, the present invention will be described in detail with accompanying exemplary embodiments. However, the exemplary embodiments disclosed herein are only for illustrative purposes and should not be construed as limiting the scope of the present invention.

### Example 1: Confirmation of concentration of target DNA using a target-specific nuclease

In order to confirm whether or not it is possible to concentrate target DNA using a target-specific nuclease, concentration of the target DNA using an RNA-guided engineered nuclease (RGEN), which is a representative example of target-specific nucleases, was confirmed.

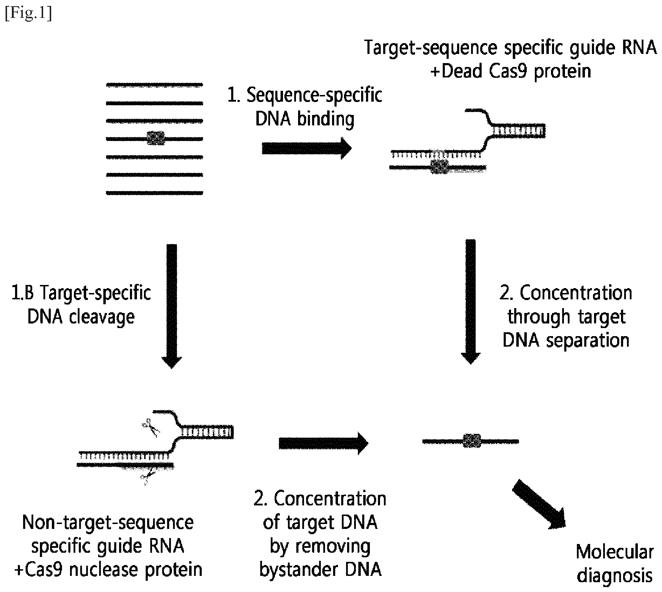

A schematic diagram thereof is shown in FIG. 1.

That is, as show in FIG. 1, it was confirmed that the genome containing the mutated DNA sequence (boxed portion) that is not normal DNA was subjected to the following two methods to concentrate target DNA.
1) Method for capturing mutated DNA by masking
   A method for concentrating by amplifying target DNA, by masking the mutated DNA for protection from random cleavage by a restriction enzyme, or by capturing the mutated DNA, using RGEN composed of a target-sequence-specific guide RNA (gRNA) and an inactivated Cas9 nuclease protein (a dead Cas9 protein or an inactivated Cas9 protein)
2) Method for amplifying after removing the normal DNA
   A method for concentrating the target DNA by amplification, after the normal DNA was cleaved to remove with RGEN composed of a non-target-sequence-specific guide RNA and a Cas9 nuclease protein.

### Example 2: Observation of a very small amount of mutation induced by gene scissors

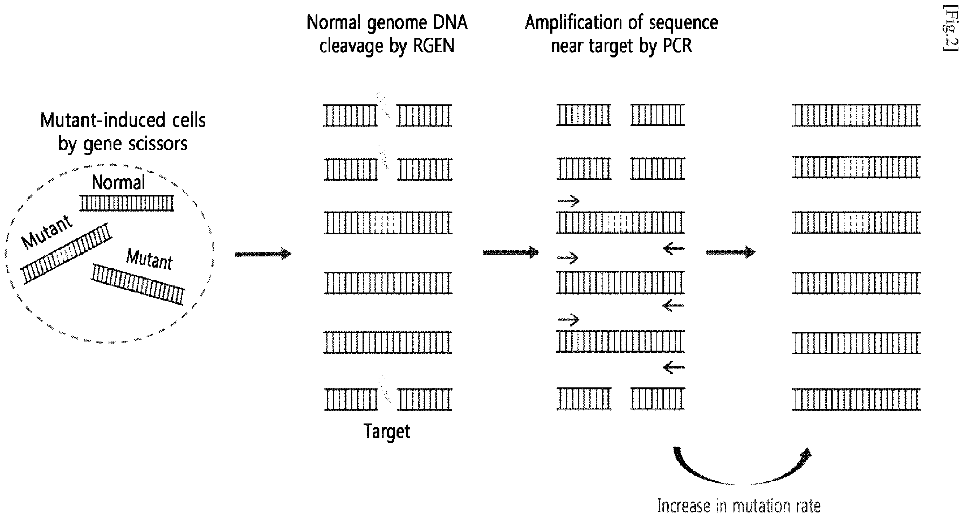

When the mutation rate induced by RGEN at intracellular targets or similar sequences of the target is very low, the process of increasing the ratio of mutant DNA to normal DNA is necessary in order to make a detection. For this, after genomic DNA was separated from a cell treated with RGEN, only the normal genomic DNA was excised by treating with the RGEN, which recognizes only the normal sequence of the target, leaving the mutant genomic DNA (FIG. 2). Subsequently, by amplifying a sequence around the target by PCR, the ratio of a PCR product amplified from the non-excised mutant genomic DNA to a PCR product amplified from the excised normal genomic DNA get relatively increased.

Specifically, after inducing a mutation by treating a cell inside with RGEN, which cleaves the *VEGFA* gene, the genomic DNA of the cell was separated, and the normal DNA was excised using each RGEN corresponding to sequences similar to the target to be observed for the presence of a mutation. After amplifying the neighboring region of the excised sequence by PCR, the sequence was read by a sequencing machine, and the ratio of the normal DNA to the mutant DNA was observed. As a result, an increase in the mutation rate of up to 1.4 to 30 times was observed compared to a case without cleaving with the RGEN (Table. 1).

**[Table 1]**

| Sequence number | RGEN target sequence | Target name | Mock_uncut mutation (%) | Mock_cut mutation (%) | RGEN_uncut mutation (%) | RGEN_cut mutation (%) | The rate of increase of mutation |
|---|---|---|---|---|---|---|---|
| Sequence number 1 | GGGGAGGGGA AGTTTGCTCCT GG | VEGFA_ Off3 | 0.04% | 0.00% | 69.15% | 95.04 % | 1.4 |
| Sequence number 2 | CGGGGGAGGG AGTTTGCTCCT GG | VEGFA_Off5 | 0.00% | 0.01% | 2.20% | 35.10 % | 26.9 |
| Sequence number 3 | GGAGGAGGGG AGTCTGCTCC AGG | VEGFA_ Off27 | 0.00% | 0.00% | 5.99% | 79.88 % | 13.3 |
| Sequence number 4 | GGTGGGGGTG GGTTTGCTCCT GG | VEGFA_ Off16 | 0.00% | 0.00% | 1.16% | 29.98 % | 29.7 |
| Sequence number 5 | GAGTGGGTGG AGTTTGCTACA GG | VEGFA_ Off15 | 0.00% | 0.00% | 2.30% | 17.12 % | 7.4 |
| Sequence number 6 | AAGTAAGGGA AGTTTGCTCCT GG | VEGFA_ Off72 | 0.00% | 0.00% | 2.92% | 54.73 % | 18.7 |

(Mock_uncut: Sample in which gDNA of normal cells was not cleaved; Mock_cut: Sample in which gDNA of normal cells was cleaved; RGEN-uncut: Sample in which gDNA of mutant induced cells by RGEN was not cleaved; and RGEN_cut: Sample in which gDNA of mutant induced cells by RGEN was cleaved)

Next, to determine how much increase could be seen in mutant sequences at a very low mutation rate, RGEN corresponding to the *FANCF* gene was applied to cells, and a genomic DNA mixture containing 54% mutation was obtained.

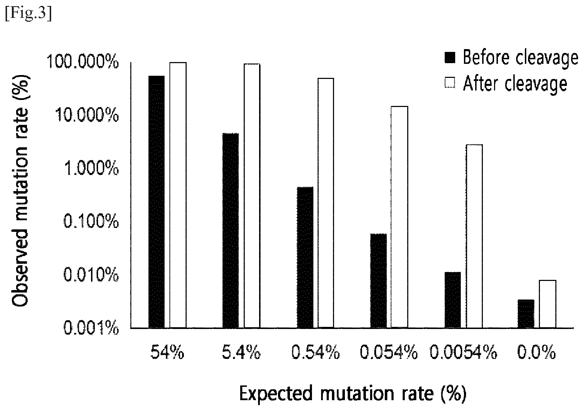

Further, after the mutant genomic DNA mixture was mixed with an amount of the normal genomic DNA, increased incrementally by powers of 10 (*i.e.,* 10, 100, 1000, etc.), a genomic DNA mixture containing the mutants at a ratio of 0.0054% to 54% was obtained, which was then cleaved with the RGEN, which cleaves only normal sequences of the *FANCF* gene. As a result, in all ranges of the mutation rates, the mutation rate was increased up to 520 times (2.8%/0.0054%) after cleavage, and this increasing effect was confirmed (FIG. 3).

The target base sequences and the sequences of sgRNA used in the above experiment are summarized in Table 2.

**[Table 2]**

| Target name | Target base sequence | RNAbase sequence (5'-> 3') |
|---|---|---|
| VEGF-A_Off3 | GGGGAGGGGAAGTT TGCTCCTGG(Sequence number 7) | 5'GGGGAGGGGAAGTTTGCTCCGUUUUAGA GCUAGAAAUAGCAAGUUAAAAUAAGGCU AGUCCGUUAUCAACUUGAAAAAGUGGCAC CGAGUCGGUGCUUUUU 3' (Sequence number 15) |
| VEGFA_Off5 | GGAGGAGGGGAGTC TGCTCCAGG(Sequence number 8) | 5'GGAGGAGGGGAGTCTGCTCCGUUUUAGA GCUAGAAAUAGCAAGUUAAAAUAAGGCU AGUCCGUUAUCAACUUGAAAAAGUGGCAC CGAGUCGGUGCUUUUU 3' (Sequence number 16) |
| VEGFA_Off27 | GGTGGGGGTGGGTTT GCTCCTGG(Sequence number 9) | 5'GGTGGGGGTGGGTTTGCTCCGUUUUAGA GCUAGAAAUAGCAAGUUAAAAUAAGGCU AGUCCGUUAUCAACUUGAAAAAGUGGCAC CGAGUCGGUGCUUUUU 3' (Sequence number 17) |
| VEGFA_Off16 | GAGTGGGTGGAGTTT GCTACAGG(Sequence number 10) | 5'GAGTGGGTGGAGTTTGCTACGUUUUAGA GCUAGAAAUAGCAAGUUAAAAUAAGGCU AGUCCGUUAUCAACUUGAAAAAGUGGCAC CGAGUCGGUGCUUUUU 3' (Sequence number 18) |
| VEGFA_Off15 | AGGTGGTGGGAGCT TGTTCCTGG(Sequence number 11) | 5'GAGGTGGTGGGAGCTTGTTCCGUUUUAG AGCUAGAAAUAGCAAGUUAAAAUAAGGC UAGUCCGUUAUCAACUUGAAAAAGUGGC ACCGAGUCGGUGCUUUUU 3' (Sequence number 19) |
| VEGFA_Off56 | GGGCAAGGGGAGGT TGCTCCTGG(Sequence number 12) | 5'GGGCAAGGGGAGGTTGCTCCGUUUUAGA GCUAGAAAUAGCAAGUUAAAAUAAGGCU AGUCCGUUAUCAACUUGAAAAAGUGGCAC CGAGUCGGUGCUUUUU 3' (Sequence number 20) |
| VEGFA_Off72 | AAGTAAGGGAAGTT TGCTCCTGG(Sequence number 13) | 5'GAAGTAAGGGAAGTTTGCTCCGUUUUAG AGCUAGAAAUAGCAAGUUAAAAUAAGGC UAGUCCGUUAUCAACUUGAAAAAGUGGC ACCGAGUCGGUGCUUUUU 3' (Sequence number 21) |
| FANCF | GGAATCCCTTCTGCA GCACCTGG(Sequence number 14) | 5'GGAATCCCTTCTGCAGCACCGUUUUAGA GCUAGAAAUAGCAAGUUAAAAUAAGGCU AGUCCGUUAUCAACUUGAAAAAGUGGCAC CGAGUCGGUGCUUUUU 3' (Sequence number 22) |

### Example 3: Confirmation of diagnosis of mutation occurring at a high frequency in oncogenes

Common oncogenes have mutations, unlike normal DNA, which are wild type. Fragments having mutations of such oncogenes are difficult to observe because the fragments are mixed at a ratio of less than 1%, generally less than 0.01% to 0.1%, in a sample, and thus it is difficult to make an early diagnosis of cancer.

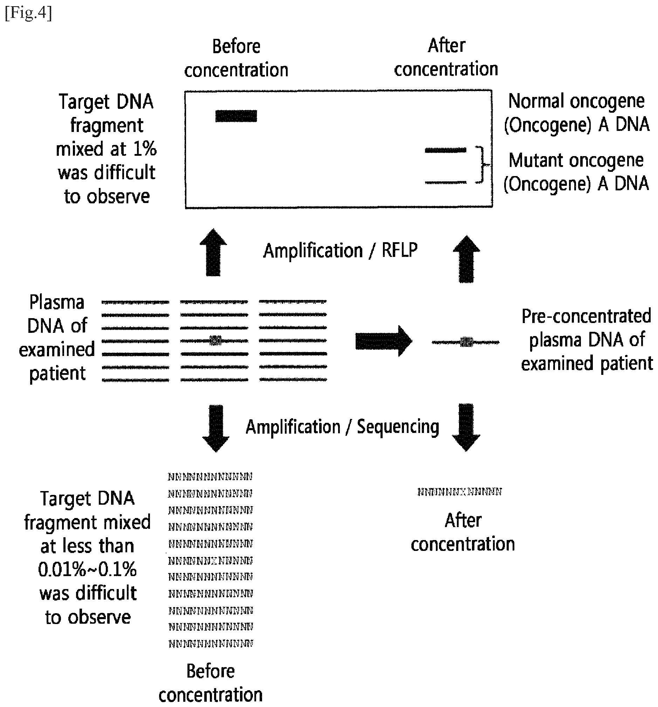

As a response, a method for determining whether or not it is possible to perform a mutation diagnosis of such oncogenes by applying the new paradigm of the present invention was shown in FIG. 4 as a schematic diagram.

That is, in order to confirm the superiority of the method of the new paradigm of the present invention for detecting the presence of the oncogene in which mutations in the blood plasma DNA of an examined patient were present, the restriction fragment length polymorphism (RFLP) and sequence analysis were performed using a control group (before concentration), in which normal DNA was not cleaved, and a test group (after concentration), in which only mutant oncogene fragments having mutations were amplified after cleaving the normal DNA with RGEN. In the RFLP analysis, it was confirmed that it was difficult to observe the target DNA fragments mixed at a ratio of less than 1% in the control group. However, in the test group, the mutant oncogene, which was a target DNA fragment, could be easily detected.

In addition, although the results from sequence analysis indicated that it was difficult to observe target DNA fragments mixed at a ratio of less than 0.01% to 0.1% in the control group, it was confirmed that because in the test group, sequence analysis could be accurately performed only for the target DNA fragment, it was easy to make an observation.

### Example 4: Preparation of RGEN for RFLP for detecting oncogene mutations

The present inventors attempted to prepare RGEN for RFLP for detecting oncogene mutations.

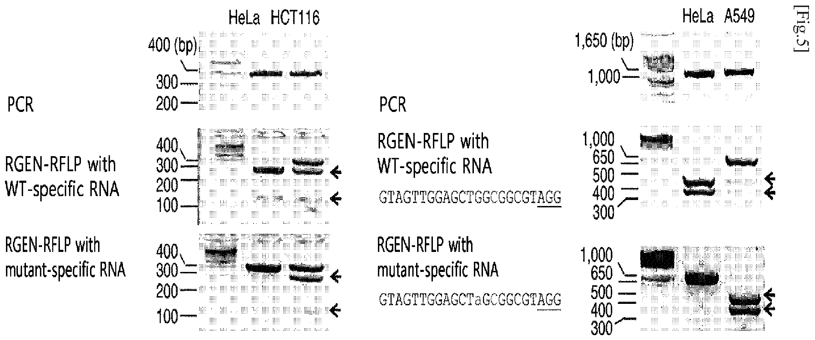

For example, RGEN specific to normal DNA and mutation-specific RGEN specific to a mutation at the 12^{th} amino acid position, which is a mutant hotspot of the K-RAS oncogene that mutates at a high frequency in a variety of cancers, were developed (FIG. 5).

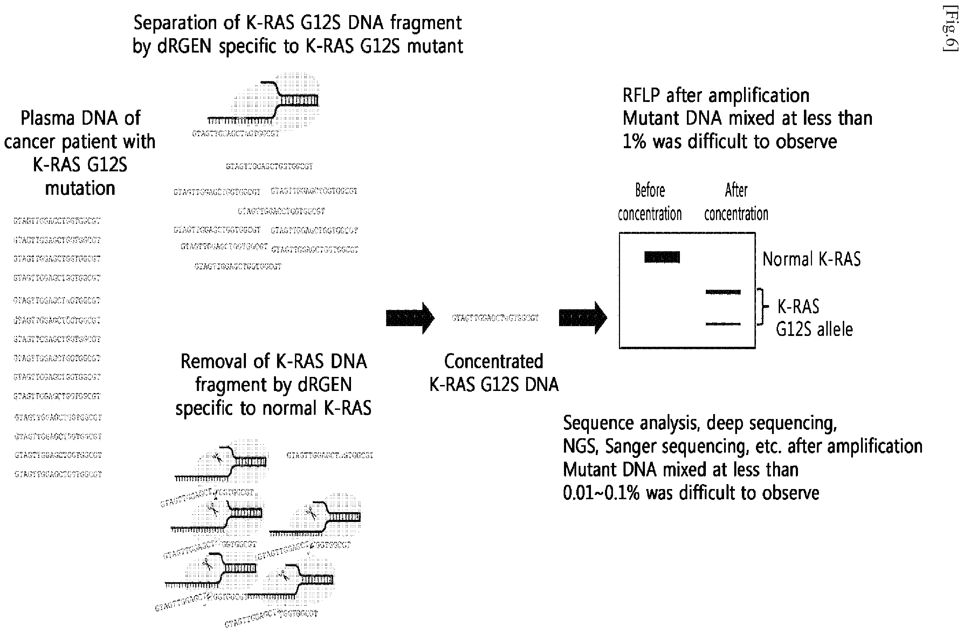

FIG. 6 shows the result of confirming through experiments whether the two methods of Example 1 can be applied to the blood plasma DNA of cancer patients having the K-RAS G123 mutation. As shown in FIG. 6, it was confirmed that the K-RAS G12S mutation was accurately detected without false negatives, etc.

### Example 5: Development of high sensitivity observation technique for a very small amount of a ctDNA mutation using RGEN

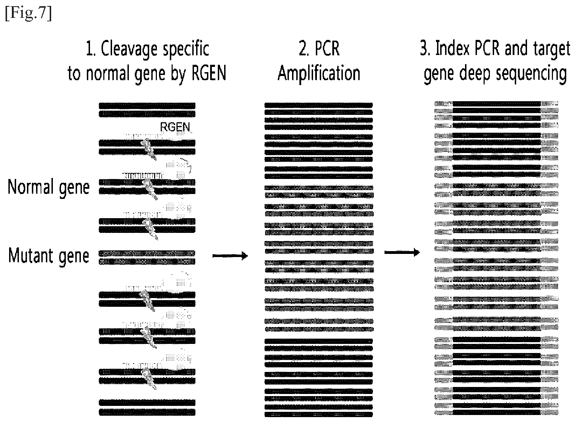

Next, circulating tumor DNA (ctDNA), which is a cancer-specific marker, was targeted for an early diagnosis of cancer. Since ctDNA in early cancer patients is mixed with normal genes and is present in a very small amount in blood, ctDNA present in a very small amount of cell-free DNA circulating in a body fluid was amplified and detected. An RNA guided endonuclease (RGEN), which specifically functions only on normal genes, was prepared and applied so that only the normal genes were excised, and the mutant genes were amplified relative to the normal genes through PCR, leaving the DNA containing frequently occurring variations in oncogenes. Afterwards, by using base sequence information obtained from performing Index PCR and targeted deep sequencing, it was possible to calculate the increased ratio of the mutant gene to the normal gene (FIG. 7).

First, in order to confirm the possibility of detecting a mutant gene mixed with a high concentration of normal gene at by CUT-PCR, a plasmid containing a mutant gene (KRAS c.35G>A, c.35G>T) of the KRAS gene, which has a high frequency of occurrence in colon cancer, and a plasmid containing a normal gene (KRAS c.35G) were each prepared and mixed at various ratios, and while the mutant gene was diluted to a smaller ratio (up to 1/1000 level) compared to the normal gene, CUT-PCR (PCR after treating with the RGEN specific to the normal gene) was performed. The normal KRAS gene was cleaved, and the ratio of the amplified normal KRAS gene to an internal control was calculated. Further, after the cancer-causing mutant gene with a high frequency of occurrence was diluted to a ratio of 1/1000 to the normal gene, and at the concentration thereof, an increased ratio of the mutant gene through CUT-PCR was calculated. The target base sequences of the RGEN specific to the normal gene and the base sequences of the sgRNA are shown in Table 3 below.

**[Table 3]**

| Tag name | Target base sequence | RNA base sequence (5' -> 3') |
|---|---|---|
| KRAS wildtype | AAACTTGTGGTAGTT GGAGCTGG (Sequence number 23) | 5'GGAAACTTGTGGTAGTTGGAGCGUUUU AGAGCUAGAAAUAGCAAGUUAAAAUAA GGCUAGUCCGUUAUCAACUUGAAAAAGU GGCACCGAGUCGGUGCUUUUUU 3' (Sequence number 24) |

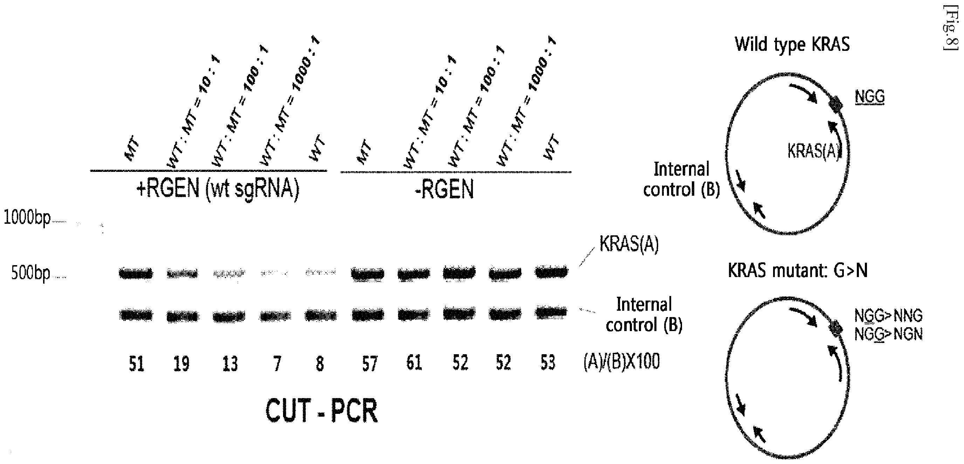

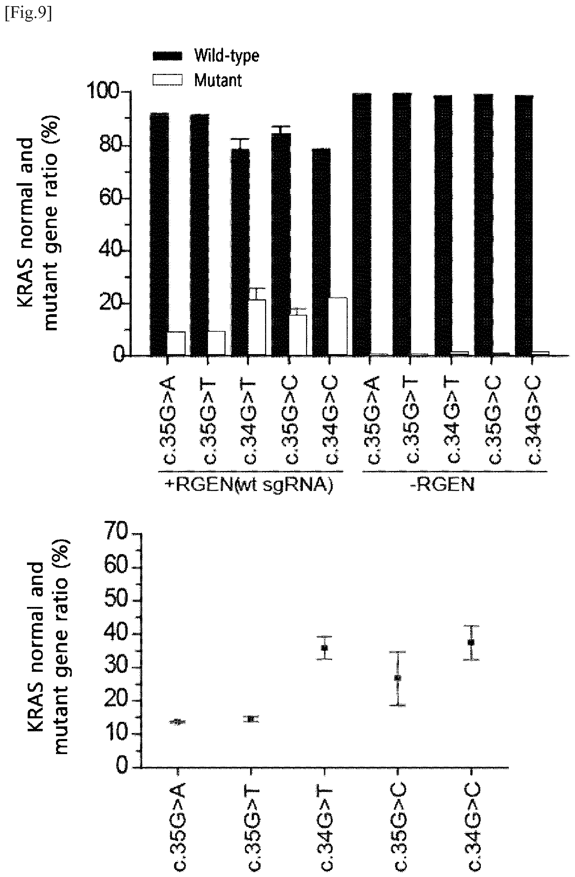

As a result, when the KRAS gene was amplified compared to the product amplified with a control group primer, it was confirmed that the RGEN specifically prepared for the normal gene functioned properly on the normal gene (FIG. 8). Further, when the region where variations of the amplified product occurred was analyzed by deep sequencing, the KRAS mutant gene, while diluted to a ratio of 1/1000 to the normal gene, was amplified up to 30 to 40 times and was accurately detected (FIG. 9, Table 4).

**[Table 4]**

| Comparison of variation rate (%) before and after RGEN treatment | | | | |
|---|---|---|---|---|
| RGEN target sequence | Type of variation | Variatin gene (%) before RGEN treatment | Variation gene (%) after RGEN treatment | Rate of increase of variation |
| AAACTTGTGGTAGTTGGAGC**TGG** (Sequence number 25) | KRAS wildtype | 0% | 0% | ∼ |
| AAACTTGTGGTAGTTGGAGC**TGA**(Sequ ence number 26) | KRAS c.35G> A | 0.27% | 8.02% | 13.71 |
| AAACTTGTGGTAGTTGGAGC**TGT**(Sequ ence number 27) | KRAS c.35G>T | 0.14% | 8.53% | 14.64 |
| AAACTTGTGGTAGTTGGAGC**TTG**(Sequ ence number 28) | KRAS c.34G>T | 0.96% | 21.19% | 35.78 |
| AAACTTGTGGTAGTTGGAGC**TGC**(Sequ ence number 29) | KRAS c.35G>C | 0.62% | 15.08% | 26.69 |
| AAACTTGTGGTAGTTGGAGC**TCG**(Sequ ence number 30) | KRAS c.34G>C | 0.93% | 21.57% | 37.41 |

<The base sequence information of the top 5 single base substitution variations having the highest frequency of occurrence among colon cancer variations, and the target base sequence information of sgRNA, which can cleave only normal genes using the variations of regions recognized as PAM, are shown. The rate of increase of each variation, before and after treating the RGEN specific to the normal gene, was calculated. Bold: PAM base sequence; underlined: Variation caused by substitution>

After the plasmid validation, the experiment for detecting ctDNA, which contains a cancer-specific gene variation, was also performed in cell-free DNA (cfDNA) obtained from human blood plasma. Since ctDNA is contained in the blood plasma in a very small amount, the mutant gene was amplified by repeating a CUT-PCR process (Treatment of the RGEN specific to the normal gene (Table 5) and PCR amplification). As a result, KRAS variations (c.35G>A (up to 192 times) and c.35G>T (up to 79 times)) with a higher frequency of occurrence were detected in samples obtained from colon cancer patients, compared to samples obtained from normal individuals (FIG. 10). The results of CUT-PCR amplification show a significantly increased sensitivity compared to those measured by existing pyrosequencing methods (Table 5).

**[Table 5]**

| Sample number | ID number | Type | Disease stage | Detected variation type (CUT-PCR) | Detected variation type (Pyrosequencing) |
|---|---|---|---|---|---|
| 1 | 01-140707-1 | Colon cancer | IIIB | GGT>GTT:p.G12V | Wild type GGT |
| 2 | 01-140707-3 | Colon cancer | I | GGT>GTT:p.G12V, GGT>GAT:p.G12D | Wild type GGT |
| 3 | 01-140708-5 | Colon cancer | IIIA | GGT>GTT:p.G12V, GGT>GAT:p.G12D | Wild type GGT |
| 4 | 01-140717-2 | Colon cancer | IIIB | GGT>GTT:p.G12V, GGT>GAT:p.G12D | GGT>GCT:p.G12A |
| 5 | 01-140718-1 | Colon cancer | IIIC | GGT>GTT:p.G12V, GGT>GAT:p.G12D | Wild type GGT |
| 6 | 01-140827-3 | Colon cancer | IIIB | GGT>GTT:p.G12V | Wild type GGT |
| 7 | 01-140811-3 | Colon cancer | IIIB | GGT>GTT:p.G12V, GGT>GAT:p.G12D | Wild type GGT |
| 8 | 01-140812-5 | Colon cancer | IIA | Wild type GGT | Wild type GGT |
| 9 | 01-141016-1 | Normal individual | None | Wild type GGT | Wild type GGT |
| 10 | 01-141016-9 | Normal individual | None | Wild type GGT | Wild type GGT |

### Example 6: Concentration of target DNA using a dCAS9:gRNA complex

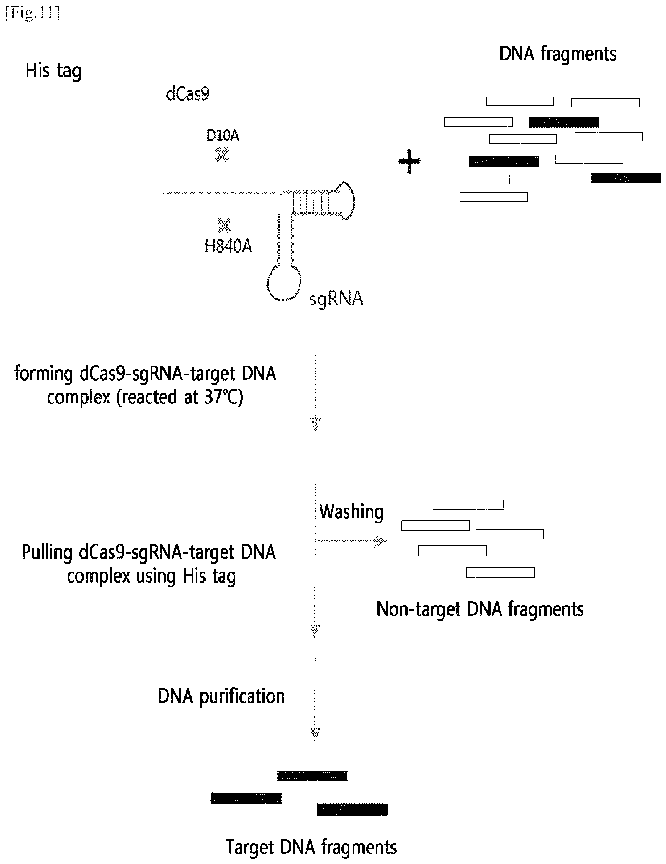

In the present invention, in order to purify particular genotype DNA, inactivated RGEN (a dCas9:gRNA complex) composed of a guide RNA and an inactivated Cas9 nuclease protein was used. The dCas9 protein has a histidine tag (a His tag) for purification, and by using a Ni-NTA magnetic bead that selectively binds to the His tag, only the dCas9 protein can be selectively purified. Further, only desired target DNA can be selectively purified by using the property of a dCas9-protein-sgRNA complex, which does not have a nuclease activity capable of specifically binding to the base sequence of DNA (FIG. 11).

### Example 6-1. Elective separation of plasmid fragments

In order to confirm that it was possible to separate only desired target DNA through inactivated RGEN (a dCas9:gRNA complex) composed of a guide RNA and an inactivated Cas9 nuclease protein, a plasmid (pUC19) was digested with restriction enzymes (Spel, Xmal, and Xhol) so that the plasmid could be distinguished by size, and plasmid DNA fragments having sizes of 4134 bp, 2570 bp, and 1263 bp were prepared.

Further, two sgRNAs were prepared for each of the plasmid DNA fragments cleaved in the above procedure (4134bp_sg#1, 4134bp_sg#2, 2570bp_sg#1, 2570bp_sg#2, 1263bp_sg#1, and 1263bp_sg#2), and the purification process was performed using each sgRNA corresponding to each target DNA or a combination thereof (4134bp sg#1+2, 2570bp_sg#1+2, and 1263bp_sg#1+2). Each sgRNA base sequence is shown in Table 6 below.

**[Table 6]**

| **sgRNA** | **Target base sequence** | **PAM base sequence** |
|---|---|---|
| 4134bp_sg#1 | GAGAACCAGACCACCCAGAA (Sequence number 31) | GGG |
| 4134bp_sg#2 | GGCAGCCCCGCCATCAAGAA(Sequence number 32) | GGG |
| 2570bp_sg#1 | GTAAGATGCTTTTCTGTGAC(Sequence number 33) | TGG |
| 2570bp_sg#2 | GATCCTTTGATCTTTTCTAC(Sequence number 34) | GGG |
| 1270bp_sg#1 | GCCTGCAAAAAAGAAGAGAA(Sequence number 35) | AGG |
| 1270bp_sg#2 | TGACATCAATTATTATACAT(Sequence number 36) | CGG |

| | | |
|---|---|---|
| * The sgRNA sequence is identical to the target base sequence, except that T is U. | | |

Then, a total of 200 µL of a mixture solution of DNA : dCas9 protein : sgRNA = 1: 20 : 100 at a molar concentration ratio thereof was mixed and prepared, followed by reacting at 37°C for 1 hour and 30 minutes. The solution was then mixed with 50 µL of Ni-NTA magnetic beads capable of specifically binding to the histidine tag, and after washing twice with 200 µL of a wash buffer, a dCas9-sgRNA-target DNA complex was purified using 200 µL of an elution buffer (Bioneer, K-7200).

Then, RNase A (Amresco, E866) was added at a concentration of 0.2 mg/mL followed by reacting at 37°C for 2 hours, and Protease K (Bioneer, 1304G) was added at a concentration of 0.2 mg/mL followed by reacting at 55°C for 45 minutes. After asgRNA and a dCas9 protein were removed, only the target DNA was purified through ethanol purification.

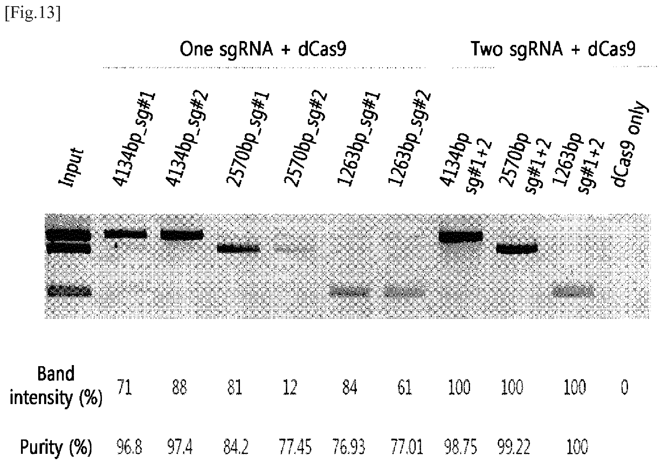

As a result, when each sgRNA corresponding to the target proteins was used separately, and when two types of sgRNAs for the target proteins were mixed and used, in all cases, it was confirmed that only the desired target DNA fragment was separated by size from the 3 DNA fragments (FIG. 13).

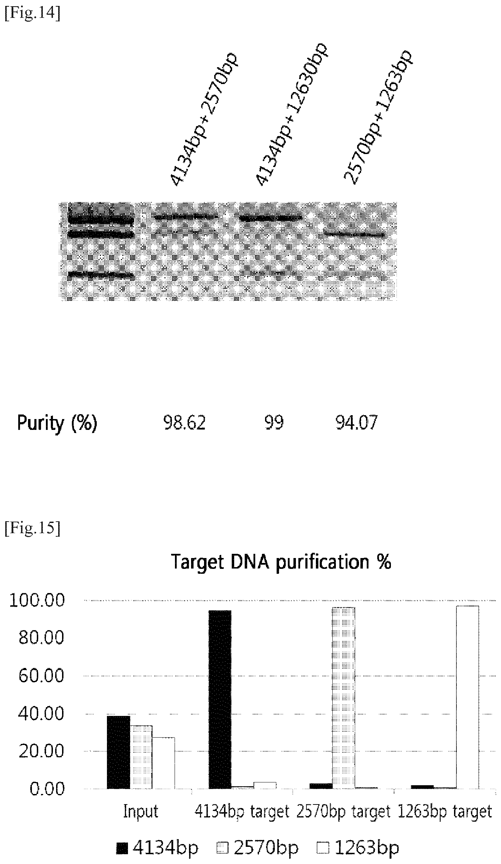

Further, when multiple target DNAs were purified at once using a total of four sgRNAs, two sgRNAs corresponding to each of the two target DNAs, it was confirmed that the target DNA corresponding to the sgRNAs used was mixed and purified (FIG. 14).

The results of purifying each plasmid DNA fragment are shown in FIG. 15. Through this, it was confirmed that each target DNA was purified to a purity of 95% or more.

### Example 6-2. Confirmation of selective separation of target exons

Next, in order to confirm that it was possible to purify an exon on the genomic DNA (gDNA) of an actual cell through inactivated RGEN (dCas9:gRNA complex) composed of a guide RNA and an inactivated Cas9 nuclease protein, gDNAs of HeLa cells and SW480 cells were extracted and cleaved into fragments 400 bp in size, and experiments for purifying the target exon were performed.

Specifically, exons of the TP53 gene of cancer cells were targeted, and three sgRNAs were used for each target exon. The sequences of each sgRNA are shown in Table 7 below. The target DNA was purified under the same conditions as in Example 6-1.

**[Table 7]**

| **sgRNA** | **Target base sequence** | **PAM** |
|---|---|---|
| Exon1_sg#1 | GGGACACTTTGCGTTCGGGC (Sequence number 37) | TGG |
| Exon1_sg#2 | AACTCTAGAGCCACCGTCCA (Sequence number 38) | GGG |
| Exon1_sg#3 | AGCGCCAGTCTTGAGCACAT (Sequence number 39) | GGG |
| Exon2&3_sg#1 | GATCCACTCACAGTTTCCAT (Sequence number 40) | AGG |
| Exon2&3_sg#2 | GTGGGAAGCGAAAATTCCAT (Sequence number 41) | GGG |
| Exon2&3_sg#3 | CTCAGAGGGGGCTCGACGCT (Sequence number 42) | AGG |
| Exon4_sg#1 | CTTCCCACAGGTCTCTGCTA (Sequence number 43) | GGG |
| Exon4_sg#2 | TGGTGGGCCTGCCCTTCCAA (Sequence number 44) | TGG |
| Exon4_sg#3 | CTTCCGGGTCACTGCCATGG (Sequence number 45) | AGG |
| Exon5_sg#1 | AGAGTTGGCGTCTACACCTC (Sequence number 46) | AGG |
| Exon5_sg#2 | GAATCAACCCACAGCTGCAC (Sequence number 47) | AGG |
| Exon5_sg#3 | CGGCACCCGCGTCCGCGCCA (Sequence number 48) | TGG |
| Exon6_sg#1 | CTCGGATAAGATGCTGAGGA (Sequence number 49) | GGG |
| Exon6_sg#2 | CACTTTTCGACATAGTGTGG (Sequence number 50) | TGG |
| Exon6_sg#3 | AAATTTGCGTGTGGAGTATT (Sequence number 51) | TGG |
| Exon7_sg#1 | GGAGTCTTCCAGTGTGATGA (Sequence number 52) | TGG |
| Exon7_sg#2 | CATGTAGTTGTAGTGGATGG (Sequence number 53) | TGG |
| Exon7_sg#3 | GCATGGGCGGCATGAACCGG (Sequence number 54) | AGG |
| Exon8_sg#1 | ACTGGGACGGAACAGCTTTG (Sequence number 55) | AGG |
| Exon8_sg#2 | GATTCTCTTCCTCTGTGCGC (Sequence number 56) | CGG |
| Exon8_sg#3 | GGTGAGGCTCCCCTTTCTTG (Sequence number 57) | CGG |
| Exon9_sg#1 | GTGAAATATTCTCCATCCAG (Sequence number 58) | TGG |
| Exon9_sg#2 | GGGAGAGGAGCTGGTGTTGT (Sequence number 59) | TGG |
| Exon10_sg#1 | TCTCGAAGCGCTCACGCCCA (Sequence number 60) | CGG |
| Exon10_sg#2 | GAACTCAAGGATGCCCAGGC (Sequence number 61) | TGG |
| Exon11_sg#1 | CAATCAGCCACATTCTAGGT (Sequence number 62) | AGG |
| Exon11_sg#2 | CTAGAACTTGACCCCCTTGA (Sequence number 63) | GGG |
| Exon11_sg#3 | GATGAAATCCTCCAGGGTGT (Sequence number 64) | GGG |

| | | |
|---|---|---|
| *The sgRNA sequence is identical to the target base sequence, except that T is U. | | |

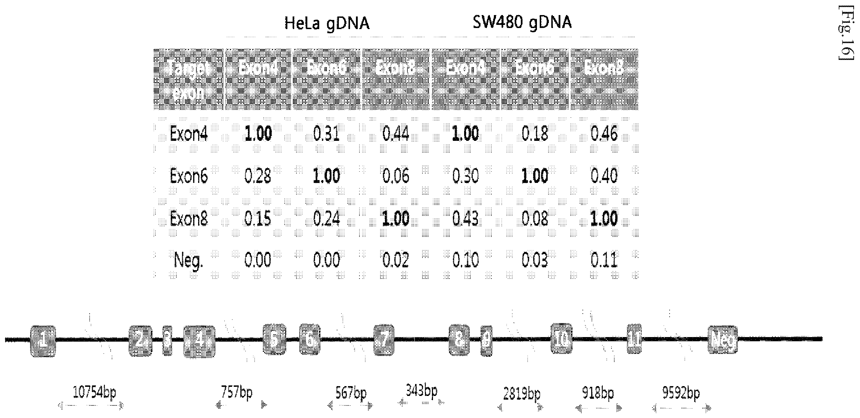

Then, in order to confirm whether the target exon was purified, real-time quantitative PCR was performed to measure the amount of the target DNA in the sample. As a result, it was confirmed that each exon was actually amplified, and it was confirmed that the target exon region was amplified up to about 2 to 20 times, compared to other exons (FIG. 16).

### Example 7: Confirmation of diagnosis by classifying pathogenic bacterial DNA of similar sequences

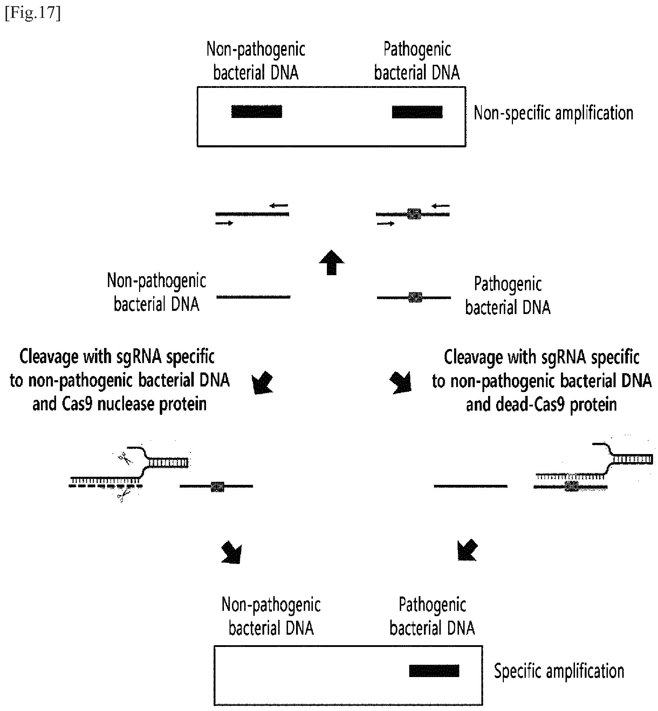

The present inventors attempted to confirm whether or not it was possible to classify sequences with a high equivalence, which are difficult to diagnose by classifying according to existing molecular diagnosis methods. For example, since a pathogenic bacterium/virus and a non-pathogenic bacterium/virus have very similar sequences, the method for confirming whether or not it was possible to make a diagnosis by classifying pathogenic and non-pathogenic bacteria by applying the new paradigm of the present invention is illustrated in FIG. 17 as a schematic diagram.

That is, when non-pathogenic bacterial DNA and pathogenic bacterial DNA are simply amplified together, non-specific amplification occurs due to sequence similarity so that non-pathogenic bacterial DNA and pathogenic bacterial DNA cannot be distinguished (top of FIG. 17).

However, by amplifying after cleaving using a guide RNA specific to non-pathogenic bacterial DNA, which causes normal reactions to subjects; and a Cas9 nuclease protein, and by using RGEN composed of a guide RNA specific to pathogenic bacterial DNA and an inactivated Cas9 nuclease protein (dead Cas9 protein), when an amplification by capturing the pathogenic bacterial DNA was performed, it was confirmed that only the pathogenic bacterial DNA, which is not easily distinguished because of having similar sequences, could be specifically amplified (bottom of FIG. 17).

The above results suggest that the new method for analyzing a genotype of the present invention, specifically, in which RGEN, unlike existing methods for analyzing a genotype, removes normal DNA in a sample by cleaving the normal DNA, and then only target DNA is amplified, or only the target DNA is captured and amplified, can accurately analyze without false positives/negatives, unlike existing PCR methods, etc.

From the foregoing, those skilled in the art to which the present invention pertains will be able to understand that the present invention may be embodied in other specific forms without modifying the technical concepts or essential characteristics of the present invention. In this regard, the exemplary embodiments disclosed herein are only for illustrative purposes and should not be construed as limiting the scope of the present invention. On the contrary, the present invention is intended to cover not only the exemplary embodiments but also various alternatives, modifications, equivalents, and other embodiments that may be included within the spirit and scope of the present invention as defined by the appended claims.

## Claims

1. A method for analyzing a genotype comprising:
(i) removing particular genotype DNA in an isolated sample, in which two or more genotypes of DNA are mixed, by cleaving the particular genotype DNA using a nuclease specific to the particular genotype DNA; and
(ii) analyzing other genotype DNA present in the isolated sample, in which the particular genotype DNA has been removed.

2. The method of claim 1, comprising:
(a) removing the particular genotype DNA in the isolated sample, in which two or more genotypes of DNA are mixed, by cleaving the particular genotype DNA, using the nuclease specific to the particular genotype DNA;
(b) amplifying other genotype DNA present in the isolated sample, in which the particular genotype DNA has been removed; and
(c) analyzing the amplified other genotype DNA.

3. The method of claim 1, wherein the nuclease is selected from the group consisting of a zinc finger nuclease (ZFN), a transcription activator-like effector nuclease (TALEN), and an RNA-guided engineered nuclease (RGEN).

4. The method according to claim 1, which is for diagnosis of cancer, wherein:
step (i), in which the particular genotype DNA is wild type DNA, is to remove the wild type DNA by cleaving the wild type DNA using the nuclease specific to the wild type DNA in the isolated sample; and
step (ii), in which other genotype DNA is DNA having a cancer-specific mutation, is to analyze the cancer-specific mutant DNA in the isolated sample, in which the wild type DNA is removed.

5. The method of claim 4, wherein the diagnosis of cancer is an early diagnosis of cancer.

6. The method of claim 4, wherein the diagnosis of cancer is predicting the prognosis of cancer.

7. The method of claim 4, wherein the isolated sample, in which two or more genotypes of DNA are mixed, is a blood sample isolated from a subject suspected of having cancer.

8. The method of claim 1, which is for predicting the prognosis of a subject that has received cell or organ transplantation, wherein:
step (i), in which the particular genotype DNA is the DNA of a subject that has received cell or organ transplantation, is to remove the DNA of the subject in a sample, which is isolated from the subject that has received cell or organ transplantation and comprises two or more genotypes of DNA, by cleaving the DNA of the subject using a nuclease specific to the DNA of the subject; and
step (ii), in which other genotype DNA is the DNA of a transplanted cell or organ, comprises analyzing the DNA of the transplanted cell or organ in the sample in which the DNA of the subject has been removed.

9. The method for claim 1, which is collecting information regarding a crime scene, wherein:
step (i), in which the particular genotype DNA is the DNA of a victim, is to remove the DNA of the victim in an isolated DNA sample, which is derived from the crime scene and comprises two or more genotypes of DNA, by cleaving the DNA of the victim using a nuclease specific to the DNA of the victim; and
step (ii), in which other genotype DNA is the DNA of an assailant, comprises analyzing the DNA of the assailant in the sample in which the DNA of the victim has been removed.

10. The method of claim 1, which is for collecting information regarding a crime scene, wherein:
step (i), in which the particular genotype DNA is the DNA of an assailant, is to remove the DNA of the assailant in an isolated DNA sample, which is derived from the crime scene and comprises two or more genotypes of DNA, by cleaving the DNA of the assailant using a nuclease specific to the DNA of the assailant; and
step (ii), in which other genotype DNA is the DNA of a victim, comprises analyzing the DNA of the victim in the sample in which the DNA of the assailant has been removed.

11. A method for analyzing a genotype comprising:
(i) removing other genotype DNA by cleaving the other genotype DNA while masking particular genotype DNA in an isolated sample, in which two or more genotypes of DNA are mixed, for protection from cleavage by RGEN capable of recognizing the other genotype DNA, using a guide RNA (gRNA) specific to the particular genotype DNA and an inactivated Cas nuclease protein (dCas); and
(ii) analyzing the particular genotype DNA present in the sample, in which the other genotype DNA has been removed.

12. The method of claim 11, comprising:
(a) removing other genotype DNA by cleaving the other genotype DNA while masking the particular genotype DNA in an isolated sample, in which two or more genotypes of DNA are mixed, for protection from cleavage by RGEN capable of recognizing the other genotype DNA, using a guide RNA (gRNA) specific to the particular genotype DNA and an inactivated Casnuclease protein (dCas);
(b) amplifying the particular genotype DNA in a sample, in which the other genotype DNA has been removed; and
(c) analyzing the amplified particular genotype DNA.

13. The method of claim 11, which is for diagnosing cancer, wherein:
step (i), in which other genotype DNA is wild type DNA, and particular DNA is DNA having a cancer-specific mutation, is to remove the wild type DNA in an isolated sample by cleaving the wild type DNA while masking the particular genotype DNA for protection from cleavage by RGEN specific to the wild type DNA, using a guide RNA capable of specifically binding to the particular DNA having the cancer-specific mutation and an inactivated Cas nuclease protein (dCas); and
step (ii) is to analyze the DNA having the cancer-specific mutation present in the
isolated sample, in which the wild type DNA has been removed.

14. The method of claim 13, wherein the diagnosing cancer is an early diagnosis of cancer.

15. The method of claim 13, wherein the diagnosing cancer is predicting the prognosis of cancer.

16. The method of claim 13, wherein the isolated sample, in which two or more genotypes of DNA are mixed, is a blood sample isolated from a subject suspected of having cancer.

17. The method of claim 11, which is for predicting the prognosis of a subject that has received cell or organ transplantation, wherein:
step (i), in which other genotype DNA is the DNA of a subject that has received cell or organ transplantation, and the particular genotype DNA is the DNA of a transplanted cell or organ, is to remove the DNA of the subject that has received cell or organ transplantation in an isolated sample by cleaving the DNA of the subject that has received cell or organ transplantation while masking the particular genotype DNA for protection from cleavage by RGEN specific to the DNA of the subject that has received cell or organ transplantation, using a guide RNA capable of specifically binding to the DNA of the transplanted cell or organ and an inactivated Cas nuclease protein (dCas); and
step (ii) is to analyze the DNA of the transplanted cell or organ present in the sample in which the DNA of the subject that has received the cell or organ transplantation has been removed.

18. The method of claim 11, which is for collecting information regarding a crime scene, wherein:
step (i), in which other genotype DNA is the DNA of a victim, and the particular genotype DNA is the DNA of an assailant, is to remove the DNA of the victim in an isolated sample by cleaving the DNA of the victim while masking the particular genotype DNA for protection from cleavage by RGEN specific to the DNA of the victim, using a guide RNA capable of specifically binding to the DNA of the assailant and an inactivated Cas nuclease protein (dCas); and
step (ii) is to analyze the DNA of the assailant present in the sample in which the DNA of the victim has been removed.

19. The method of claim 11, which is for collecting information regarding a crime scene, wherein:
step (i), in which other genotype DNA is the DNA of an assailant, and the particular genotype DNA is the DNA of a victim, is to remove the DNA of the assailant in an isolated sample by cleaving the DNA of the assailant while masking the particular genotype DNA for protection from cleavage by RGEN specific to the DNA of the assailant, using a guide RNA capable of specifically binding to the DNA of the victim and an inactivated Cas nuclease protein (dCas); and
step (ii) is to analyze the DNA of the victim present in the sample, in which the DNA of the assailant has been removed.

20. The method of claim 11, wherein the inactivated Cas protein lacks DNA cleavage activity of Cas proteins.

21. The method of claim 20, wherein the inactivated Cas protein is a variant of a Cas9 protein having a D10A, H840A, or D10A/H840A mutation.

22. A method for analyzing a genotype of DNA in an isolated sample comprising:
(i) removing DNA of a non-pathogenic bacterium or virus in the isolated sample by cleaving the DNA of the non-pathogenic bacterium or virus by treating the DNA of the non-pathogenic bacterium or virus with a nuclease specific to the DNA of the non-pathogenic bacterium or virus, in the isolated sample having DNA of bacteria or viruses; and
(ii) analyzing DNA of a pathogenic bacterium or virus in the isolated sample in which the DNA of the non-pathogenic bacterium or virus has been removed.

23. The method of claim 22, comprising:
(a) removing the DNA of the non-pathogenic bacterium or virus by cleaving the DNA of the non-pathogenic bacterium or virus by treating the DNA of the non-pathogenic bacterium or virus with a nuclease specific to the DNA of the non-pathogenic bacterium or virus, in the isolated sample having the DNA of bacteria or viruses;
(b) amplifying the DNA of a pathogenic bacterium or virus in the isolated sample in which the DNA of the non-pathogenic bacterium or virus has been removed; and
(c) analyzing the amplified DNA of the pathogenic bacterium or virus.

24. The method of claim 22, wherein the nuclease is selected from the group consisting of a zinc finger nuclease (ZFN), a transcription activator-like effector nuclease (TALEN), and an RNA-guided engineered nuclease (RGEN).

25. A method for analyzing a genotype of DNA in an isolated sample comprising:
(i) removing DNA of a non-pathogenic bacterium or virus in the isolated sample by cleaving the DNA of the non-pathogenic bacterium or virus while masking DNA of a pathogenic bacterium or virus for protection from cleavage by RGEN specific to the non-pathogenic bacterium or virus, using a guide RNA specific to the pathogenic bacterium or virus and an inactivated Cas nuclease protein (dCas); and
(ii) analyzing the DNA of the pathogenic bacterium or virus in the isolated sample in which the DNA of the non-pathogenic bacterium or virus has been removed.

26. The method of claim 25, comprising:
(a) removing the DNA of the non-pathogenic bacterium or virus in the isolated sample by cleaving the DNA of the non-pathogenic bacterium or virus while masking the DNA of a pathogenic bacterium or virus for protection from cleavage by RGEN specific to the non-pathogenic bacterium or virus, using a guide RNA specific to the DNA of the pathogenic bacterium or virus and an inactivated Cas nuclease protein (dCas);
(b) amplifying the DNA of the pathogenic bacterium or virus in the isolated sample in which the DNA of the non-pathogenic bacterium or virus has been removed; and
(c) analyzing the amplified DNA of the pathogenic bacterium or virus.
